# EUROPEAN PATENT APPLICATION

(11) **EP 2 992 892 A1**
(43) Date of publication of application: **09.03.2016**
(21) Application number: 14183699.9
(22) Date of filing: 05.09.2014
(51) Int. Cl.: A61K 38/17, C07K 14/435

(54) **Fusion protein for use in the treatment of mitochondrial diseases**

(71) Applicant: Universität zu Köln, 50923 Köln (DE)
(72) Inventor: Wenz, Tina, 50823 Köln (DE); Tischner, Christin, 50939 Köln (DE)
(74) Representative: Michalski Hüttermann & Partner Patentanwälte mbB

(57) **Abstract**

The present invention relates to a fusion protein comprising a protein transduction domain, a mitochondrial targeting sequence, and a mitochondrial elongation factor G1 peptide, and to a use in the treatment of a mitochondrial disease.

SEQUENCE LISTING

<110>  Universität zu Köln

<120>  Fusion protein for use in the treatment of mitochondrial diseases

<130>  UD 40654 / SAM

<160>  48

<170>  PatentIn version 3.3

<210>  1
<211>  37
<212>  PRT
<213>  Homo sapiens

<400>  1

Met Arg Leu Leu Gly Ala Ala Ala Val Ala Ala Leu Gly Arg Gly Arg
1               5                   10                  15

Ala Pro Ala Ser Leu Gly Trp Gln Arg Lys Gln Val Asn Trp Lys Ala
            20                  25                  30

Cys Arg Trp Ser Ser
        35

<210>  2
<211>  714
<212>  PRT
<213>  Homo sapiens

<400>  2

Ser Gly Val Ile Pro Asn Glu Lys Ile Arg Asn Ile Gly Ile Ser Ala
1               5                   10                  15

His Ile Asp Ser Gly Lys Thr Thr Leu Thr Glu Arg Val Leu Tyr Tyr
            20                  25                  30

Thr Gly Arg Ile Ala Lys Met His Glu Val Lys Gly Lys Asp Gly Val
        35                  40                  45

Gly Ala Val Met Asp Ser Met Glu Leu Glu Arg Gln Arg Gly Ile Thr
    50                  55                  60

Ile Gln Ser Ala Ala Thr Tyr Thr Met Trp Lys Asp Val Asn Ile Asn
65                  70                  75                  80

Ile Ile Asp Thr Pro Gly His Val Asp Phe Thr Ile Glu Val Glu Arg
                85                  90                  95

Ala Leu Arg Val Leu Asp Gly Ala Val Leu Val Leu Cys Ala Val Gly

            100                 105                 110

Gly Val Gln Cys Gln Thr Met Thr Val Asn Arg Gln Met Lys Arg Tyr
        115                 120                 125

Asn Val Pro Phe Leu Thr Phe Ile Asn Lys Leu Asp Arg Met Gly Ser
    130                 135                 140

Asn Pro Ala Arg Ala Leu Gln Gln Met Arg Ser Lys Leu Asn His Asn
145                 150                 155                 160

Ala Ala Phe Met Gln Ile Pro Met Gly Leu Glu Gly Asn Phe Lys Gly
                165                 170                 175

Ile Val Asp Leu Ile Glu Glu Arg Ala Ile Tyr Phe Asp Gly Asp Phe
            180                 185                 190

Gly Gln Ile Val Arg Tyr Gly Glu Ile Pro Ala Glu Leu Arg Ala Ala
        195                 200                 205

Ala Thr Asp His Arg Gln Glu Leu Ile Glu Cys Val Ala Asn Ser Asp
    210                 215                 220

Glu Gln Leu Gly Glu Met Phe Leu Glu Glu Lys Ile Pro Ser Ile Ser
225                 230                 235                 240

Asp Leu Lys Leu Ala Ile Arg Arg Ala Thr Leu Lys Arg Ser Phe Thr
                245                 250                 255

Pro Val Phe Leu Gly Ser Ala Leu Lys Asn Lys Gly Val Gln Pro Leu
            260                 265                 270

Leu Asp Ala Val Leu Glu Tyr Leu Pro Asn Pro Ser Glu Val Gln Asn
        275                 280                 285

Tyr Ala Ile Leu Asn Lys Glu Asp Asp Ser Lys Glu Lys Thr Lys Ile
    290                 295                 300

Leu Met Asn Ser Ser Arg Asp Asn Ser His Pro Phe Val Gly Leu Ala
305                 310                 315                 320

Phe Lys Leu Glu Val Gly Arg Phe Gly Gln Leu Thr Tyr Val Arg Ser
                325                 330                 335

Tyr Gln Gly Glu Leu Lys Lys Gly Asp Thr Ile Tyr Asn Thr Arg Thr
            340                 345                 350

Arg Lys Lys Val Arg Leu Gln Arg Leu Ala Arg Met His Ala Asp Met
        355                 360                 365

Met Glu Asp Val Glu Glu Val Tyr Ala Gly Asp Ile Cys Ala Leu Phe
    370                 375                 380

Gly Ile Asp Cys Ala Ser Gly Asp Thr Phe Thr Asp Lys Ala Asn Ser
385                 390                 395                 400

Gly Leu Ser Met Glu Ser Ile His Val Pro Asp Pro Val Ile Ser Ile
                405                 410                 415

Ala Met Lys Pro Ser Asn Lys Asn Asp Leu Glu Lys Phe Ser Lys Gly
            420                 425                 430

Ile Gly Arg Phe Thr Arg Glu Asp Pro Thr Phe Lys Val Tyr Phe Asp
        435                 440                 445

Thr Glu Asn Lys Glu Thr Val Ile Ser Gly Met Gly Glu Leu His Leu
    450                 455                 460

Glu Ile Tyr Ala Gln Arg Leu Glu Arg Glu Tyr Gly Cys Pro Cys Ile
465                 470                 475                 480

Thr Gly Lys Pro Lys Val Ala Phe Arg Glu Thr Ile Thr Ala Pro Val
                485                 490                 495

Pro Phe Asp Phe Thr His Lys Lys Gln Ser Gly Gly Ala Gly Gln Tyr
            500                 505                 510

Gly Lys Val Ile Gly Val Leu Glu Pro Leu Asp Pro Glu Asp Tyr Thr
        515                 520                 525

Lys Leu Glu Phe Ser Asp Glu Thr Phe Gly Ser Asn Ile Pro Lys Gln
    530                 535                 540

Phe Val Pro Ala Val Glu Lys Gly Phe Leu Asp Ala Cys Glu Lys Gly
545                 550                 555                 560

Pro Leu Ser Gly His Lys Leu Ser Gly Leu Arg Phe Val Leu Gln Asp
                565                 570                 575

Gly Ala His His Met Val Asp Ser Asn Glu Ile Ser Phe Ile Arg Ala
            580                 585                 590

Gly Glu Gly Ala Leu Lys Gln Ala Leu Ala Asn Ala Thr Leu Cys Ile
        595                 600                 605

Leu Glu Pro Ile Met Ala Val Glu Val Val Ala Pro Asn Glu Phe Gln
    610                 615                 620

Gly Gln Val Ile Ala Gly Ile Asn Arg Arg His Gly Val Ile Thr Gly
625                 630                 635                 640

Gln Asp Gly Val Glu Asp Tyr Phe Thr Leu Tyr Ala Asp Val Pro Leu
                645                 650                 655

Asn Asp Met Phe Gly Tyr Ser Thr Glu Leu Arg Ser Cys Thr Glu Gly
            660                 665                 670

Lys Gly Glu Tyr Thr Met Glu Tyr Ser Arg Tyr Gln Pro Cys Leu Pro
        675                 680                 685

Ser Thr Gln Glu Asp Val Ile Asn Lys Tyr Leu Glu Ala Thr Gly Gln
    690                 695                 700

Leu Pro Val Lys Lys Gly Lys Ala Lys Asn
705                 710

<210>  3
<211>  25
<212>  PRT
<213>  Homo sapiens

<400>  3

Met Ser Val Leu Thr Pro Leu Leu Leu Arg Gly Leu Thr Gly Ser Ala
1               5                   10                  15

Arg Arg Leu Pro Val Pro Arg Ala Lys
            20                  25

<210>  4
<211>  24
<212>  PRT
<213>  Homo sapiens

<400>  4

Met Leu Ser Arg Ala Val Cys Gly Thr Ser Arg Gln Leu Ala Pro Val
1               5                   10                  15

Leu Gly Tyr Leu Gly Ser Arg Gln
            20

<210>  5
<211>  19
<212>  PRT
<213>  Homo sapiens

<400>  5

Met Ala Phe Leu Arg Ser Met Trp Gly Val Leu Ser Ala Leu Gly Arg
1               5                   10                  15

Ser Gly Ala

<210>  6
<211>  11
<212>  PRT
<213>  Human immunodeficiency virus

<400>  6

Tyr Gly Arg Lys Lys Arg Arg Gln Arg Arg Arg
1               5                   10

<210>  7
<211>  10
<212>  PRT
<213>  Human immunodeficiency virus

<400>  7

Gly Arg Lys Lys Arg Arg Gln Arg Arg Arg
1               5                   10

<210>  8
<211>  12
<212>  PRT
<213>  Human immunodeficiency virus

<400>  8

Gly Arg Lys Lys Arg Arg Gln Arg Arg Arg Pro Gln
1               5                   10

<210>  9
<211>  6
<212>  PRT
<213>  Artificial

<220>
<223>  tag

<400>  9

His His His His His His
1               5

<210>  10
<211>  10
<212>  PRT
<213>  Artificial

<220>
<223>  tag

<400>  10

His His His His His His His His His His
1               5                   10


<210>  11
<211>  9
<212>  PRT
<213>  Artificial

<220>
<223>  tag

<400>  11

Ala Trp Arg His Pro Gln Phe Gly Gly
1               5


<210>  12
<211>  8
<212>  PRT
<213>  Artificial

<220>
<223>  tag

<400>  12

Trp Ser His Pro Gln Phe Glu Lys
1               5


<210>  13
<211>  9
<212>  PRT
<213>  Artificial

<220>
<223>  tag

<400>  13

Tyr Pro Tyr Asp Val Pro Asp Tyr Ala
1               5


<210>  14
<211>  10
<212>  PRT
<213>  Artificial

<220>
<223>  tag

<400>  14

Glu Gln Lys Leu Ile Ser Glu Glu Asp Leu
1               5                   10

<210>  15
<211>  8
<212>  PRT
<213>  Artificial

<220>
<223>  tag

<400>  15

Asp Tyr Lys Asp Asp Asp Asp Lys
1               5


<210>  16
<211>  778
<212>  PRT
<213>  Artificial

<220>
<223>  fusion protein

<400>  16

His His His His His His Gly Met Gly Ala Ala Gly Arg Lys Lys Arg
1               5                   10                  15

Arg Gln Arg Arg Arg Pro Pro Ala Gly Thr Ser Met Arg Leu Leu Gly
            20                  25                  30

Ala Ala Ala Val Ala Ala Leu Gly Arg Gly Arg Ala Pro Ala Ser Leu
        35                  40                  45

Gly Trp Gln Arg Lys Gln Val Asn Trp Lys Ala Cys Arg Trp Ser Ser
    50                  55                  60

Ser Gly Val Ile Pro Asn Glu Lys Ile Arg Asn Ile Gly Ile Ser Ala
65                  70                  75                  80

His Ile Asp Ser Gly Lys Thr Thr Leu Thr Glu Arg Val Leu Tyr Tyr
                85                  90                  95

Thr Gly Arg Ile Ala Lys Met His Glu Val Lys Gly Lys Asp Gly Val
            100                 105                 110

Gly Ala Val Met Asp Ser Met Glu Leu Glu Arg Gln Arg Gly Ile Thr
        115                 120                 125

Ile Gln Ser Ala Ala Thr Tyr Thr Met Trp Lys Asp Val Asn Ile Asn
    130                 135                 140

Ile Ile Asp Thr Pro Gly His Val Asp Phe Thr Ile Glu Val Glu Arg

145                 150                 155                 160

Ala Leu Arg Val Leu Asp Gly Ala Val Leu Val Leu Cys Ala Val Gly
                165                 170                 175

Gly Val Gln Cys Gln Thr Met Thr Val Asn Arg Gln Met Lys Arg Tyr
            180                 185                 190

Asn Val Pro Phe Leu Thr Phe Ile Asn Lys Leu Asp Arg Met Gly Ser
        195                 200                 205

Asn Pro Ala Arg Ala Leu Gln Gln Met Arg Ser Lys Leu Asn His Asn
    210                 215                 220

Ala Ala Phe Met Gln Ile Pro Met Gly Leu Glu Gly Asn Phe Lys Gly
225                 230                 235                 240

Ile Val Asp Leu Ile Glu Glu Arg Ala Ile Tyr Phe Asp Gly Asp Phe
                245                 250                 255

Gly Gln Ile Val Arg Tyr Gly Glu Ile Pro Ala Glu Leu Arg Ala Ala
            260                 265                 270

Ala Thr Asp His Arg Gln Glu Leu Ile Glu Cys Val Ala Asn Ser Asp
        275                 280                 285

Glu Gln Leu Gly Glu Met Phe Leu Glu Glu Lys Ile Pro Ser Ile Ser
    290                 295                 300

Asp Leu Lys Leu Ala Ile Arg Arg Ala Thr Leu Lys Arg Ser Phe Thr
305                 310                 315                 320

Pro Val Phe Leu Gly Ser Ala Leu Lys Asn Lys Gly Val Gln Pro Leu
                325                 330                 335

Leu Asp Ala Val Leu Glu Tyr Leu Pro Asn Pro Ser Glu Val Gln Asn
            340                 345                 350

Tyr Ala Ile Leu Asn Lys Glu Asp Asp Ser Lys Glu Lys Thr Lys Ile
        355                 360                 365

Leu Met Asn Ser Ser Arg Asp Asn Ser His Pro Phe Val Gly Leu Ala
    370                 375                 380

Phe Lys Leu Glu Val Gly Arg Phe Gly Gln Leu Thr Tyr Val Arg Ser
385                 390                 395                 400

Tyr Gln Gly Glu Leu Lys Lys Gly Asp Thr Ile Tyr Asn Thr Arg Thr
                405                 410                 415

Arg Lys Lys Val Arg Leu Gln Arg Leu Ala Arg Met His Ala Asp Met
            420                 425                 430

Met Glu Asp Val Glu Glu Val Tyr Ala Gly Asp Ile Cys Ala Leu Phe
        435                 440                 445

Gly Ile Asp Cys Ala Ser Gly Asp Thr Phe Thr Asp Lys Ala Asn Ser
    450                 455                 460

Gly Leu Ser Met Glu Ser Ile His Val Pro Asp Pro Val Ile Ser Ile
465                 470                 475                 480

Ala Met Lys Pro Ser Asn Lys Asn Asp Leu Glu Lys Phe Ser Lys Gly
                485                 490                 495

Ile Gly Arg Phe Thr Arg Glu Asp Pro Thr Phe Lys Val Tyr Phe Asp
            500                 505                 510

Thr Glu Asn Lys Glu Thr Val Ile Ser Gly Met Gly Glu Leu His Leu
        515                 520                 525

Glu Ile Tyr Ala Gln Arg Leu Glu Arg Glu Tyr Gly Cys Pro Cys Ile
    530                 535                 540

Thr Gly Lys Pro Lys Val Ala Phe Arg Glu Thr Ile Thr Ala Pro Val
545                 550                 555                 560

Pro Phe Asp Phe Thr His Lys Lys Gln Ser Gly Gly Ala Gly Gln Tyr
                565                 570                 575

Gly Lys Val Ile Gly Val Leu Glu Pro Leu Asp Pro Glu Asp Tyr Thr
            580                 585                 590

Lys Leu Glu Phe Ser Asp Glu Thr Phe Gly Ser Asn Ile Pro Lys Gln
        595                 600                 605

Phe Val Pro Ala Val Glu Lys Gly Phe Leu Asp Ala Cys Glu Lys Gly
    610                 615                 620

Pro Leu Ser Gly His Lys Leu Ser Gly Leu Arg Phe Val Leu Gln Asp
625                 630                 635                 640

Gly Ala His His Met Val Asp Ser Asn Glu Ile Ser Phe Ile Arg Ala
                645                 650                 655

Gly Glu Gly Ala Leu Lys Gln Ala Leu Ala Asn Ala Thr Leu Cys Ile
            660                 665                 670

Leu Glu Pro Ile Met Ala Val Glu Val Val Ala Pro Asn Glu Phe Gln
        675                 680                 685

Gly Gln Val Ile Ala Gly Ile Asn Arg Arg His Gly Val Ile Thr Gly
    690                 695                 700

Gln Asp Gly Val Glu Asp Tyr Phe Thr Leu Tyr Ala Asp Val Pro Leu
705                 710                 715                 720

Asn Asp Met Phe Gly Tyr Ser Thr Glu Leu Arg Ser Cys Thr Glu Gly
                725                 730                 735

Lys Gly Glu Tyr Thr Met Glu Tyr Ser Arg Tyr Gln Pro Cys Leu Pro
            740                 745                 750

Ser Thr Gln Glu Asp Val Ile Asn Lys Tyr Leu Glu Ala Thr Gly Gln
        755                 760                 765

Leu Pro Val Lys Lys Gly Lys Ala Lys Asn
    770                 775


<210>  17
<211>  6
<212>  PRT
<213>  Artificial

<220>
<223>  linker

<400>  17

Pro Pro Ala Gly Thr Ser
1               5


<210>  18
<211>  5
<212>  PRT
<213>  Artificial

<220>
<223>  linker

<400>  18

Gly Met Gly Gly Ala
1               5


<210>  19
<211>  778

<212>  PRT
<213>  Artificial

<220>
<223>  fusion protein

<400>  19

His His His His His His Gly Met Gly Ala Ala Gly Arg Lys Lys Arg
1               5                   10                  15

Arg Gln Arg Arg Arg Pro Pro Ala Gly Thr Ser Met Arg Leu Leu Arg
            20                  25                  30

Val Ala Ala Ala Leu Gly Arg Gly Pro Phe Pro Arg Val Pro Ala Val
        35                  40                  45

Leu Gly Trp Gln Gly Lys Gln Ala Asp Trp Lys Thr Arg Arg Trp Cys
    50                  55                  60

Ser Ser Gly Pro Val Pro Asn Glu Lys Ile Arg Asn Ile Gly Ile Ser
65                  70                  75                  80

Ala His Ile Asp Ser Gly Lys Thr Thr Leu Thr Glu Arg Val Leu Tyr
                85                  90                  95

Tyr Thr Gly Arg Ile Ala Thr Met His Glu Val Lys Gly Lys Asp Gly
            100                 105                 110

Val Gly Ala Val Met Asp Ser Met Glu Leu Glu Arg Gln Arg Gly Ile
        115                 120                 125

Thr Ile Gln Ser Ala Ala Thr Tyr Thr Met Trp Lys Asp Ile Asn Ile
    130                 135                 140

Asn Ile Ile Asp Thr Pro Gly His Val Asp Phe Thr Ile Glu Val Glu
145                 150                 155                 160

Arg Ala Leu Arg Val Leu Asp Gly Ala Val Leu Val Leu Cys Ala Val
                165                 170                 175

Gly Gly Val Gln Cys Gln Thr Met Thr Val Ser Arg Gln Met Lys Arg
            180                 185                 190

Tyr Asn Val Pro Phe Leu Thr Phe Ile Asn Lys Leu Asp Arg Met Gly
        195                 200                 205

Ser Asn Pro Ser Arg Ala Leu Gln Gln Met Arg Ser Lys Leu Asn His
    210                 215                 220

Asn Ala Ala Phe Val Gln Ile Pro Ile Gly Leu Glu Gly Asp Phe Lys
225                 230                 235                 240

Gly Ile Ile Asp Leu Ile Glu Glu Arg Ala Ile Tyr Phe Asp Gly Asp
                245                 250                 255

Phe Gly Gln Ile Val Arg Tyr Asp Glu Ile Pro Ala Gly Leu Arg Ala
            260                 265                 270

Ala Ala Ala Asp His Arg Gln Glu Leu Ile Glu Cys Val Ala Asn Ser
        275                 280                 285

Asp Glu Gln Leu Gly Glu Leu Phe Leu Glu Glu Lys Ile Pro Ser Val
    290                 295                 300

Ser Asp Leu Lys Arg Ala Ile Arg Arg Ala Thr Leu Ser Arg Ser Phe
305                 310                 315                 320

Thr Pro Val Phe Leu Gly Ser Ala Leu Lys Asn Lys Gly Val Gln Pro
                325                 330                 335

Leu Leu Asp Ala Val Leu Glu Tyr Leu Pro Asn Pro Ser Glu Val Gln
            340                 345                 350

Asn Tyr Ala Ile Leu Asn Gln Asn Asp Ser Lys Glu Lys Thr Lys Ile
        355                 360                 365

Leu Met Asn Pro Gln Arg Asp Asp Ser His Pro Phe Val Gly Leu Ala
    370                 375                 380

Phe Lys Leu Glu Ala Gly Arg Phe Gly Gln Leu Thr Tyr Val Arg Asn
385                 390                 395                 400

Tyr Gln Gly Glu Leu Lys Lys Gly Ser Thr Ile Tyr Asn Thr Arg Thr
                405                 410                 415

Gly Lys Lys Val Arg Val Gln Arg Leu Val Arg Met His Ala Asp Met
            420                 425                 430

Met Glu Asp Val Glu Glu Val Tyr Ala Gly Asp Ile Cys Ala Leu Phe
        435                 440                 445

Gly Ile Asp Cys Ala Ser Gly Asp Thr Phe Thr Asn Lys Asp Asn Ser
    450                 455                 460

Asp Leu Ser Met Glu Ser Ile His Val Pro Glu Pro Val Ile Ser Ile
465                 470                 475                 480

Ala Met Arg Pro Ser Asn Lys Asn Asp Leu Glu Lys Phe Ser Lys Gly
                485                 490                 495

Ile Gly Arg Phe Thr Arg Glu Asp Pro Thr Phe Lys Val His Phe Asp
            500                 505                 510

Pro Glu Ser Lys Glu Thr Ile Val Ser Gly Met Gly Glu Leu His Leu
        515                 520                 525

Glu Ile Tyr Ala Gln Arg Met Glu Arg Glu Tyr Gly Cys Pro Cys Ile
    530                 535                 540

Thr Gly Lys Pro Lys Val Ala Phe Arg Glu Thr Ile Val Ala Pro Val
545                 550                 555                 560

Pro Phe Asp Phe Thr His Lys Lys Gln Ser Gly Gly Ala Gly Gln Phe
                565                 570                 575

Gly Lys Val Ile Gly Val Leu Glu Pro Leu Pro Pro Glu Asp Tyr Thr
            580                 585                 590

Lys Leu Glu Phe Ser Asp Glu Thr Phe Gly Ser Asn Val Pro Lys Gln
        595                 600                 605

Phe Val Pro Ala Val Glu Lys Gly Phe Leu Asp Ala Cys Glu Lys Gly
    610                 615                 620

Pro Leu Ser Gly His Lys Leu Ser Gly Leu Arg Phe Val Leu Gln Asp
625                 630                 635                 640

Gly Ala His His Met Val Asp Ser Asn Glu Ile Ser Phe Ile Arg Ala
                645                 650                 655

Gly Glu Gly Ala Leu Lys Gln Ala Leu Ala Asn Gly Thr Leu Cys Ile
            660                 665                 670

Ile Glu Pro Ile Met Ser Val Glu Val Ile Ala Pro Asn Glu Phe Gln
        675                 680                 685

Gly Thr Val Phe Ala Gly Ile Asn Arg Arg His Gly Val Ile Thr Gly
    690                 695                 700

Gln Asp Gly Ile Glu Asp Tyr Phe Thr Leu Tyr Ala Asp Val Pro Leu
705                 710                 715                 720

Asn Asn Met Phe Gly Tyr Ser Thr Glu Leu Arg Ser Cys Thr Glu Gly

725                 730                 735

Lys Gly Glu Tyr Thr Met Glu Tyr Cys Arg Tyr Gln Pro Cys Ser Pro
            740                 745                 750

Ser Thr Gln Glu Glu Leu Ile Asn Lys Tyr Leu Glu Ala Thr Gly Gln
        755                 760                 765

Leu Pro Val Lys Lys Gly Lys Ala Lys Asn
    770                 775

<210>  20
<211>  32
<212>  DNA
<213>  Artificial

<220>
<223>  primer

<400>  20
agcatgacta gtatgcggct gctgcgggtc gc                                32

<210>  21
<211>  62
<212>  DNA
<213>  Artificial

<220>
<223>  primer

<400>  21
catgctctcg agtcaagcgt aatctggtac gtcgtatggg tagttcttgg ctttcccttt      60

tt                                                                 62

<210>  22
<211>  787
<212>  PRT
<213>  Artificial

<220>
<223>  fusion protein

<400>  22

His His His His His His Gly Met Gly Ala Ala Gly Arg Lys Lys Arg
1               5                   10                  15

Arg Gln Arg Arg Arg Pro Pro Ala Gly Thr Ser Met Arg Leu Leu Arg
            20                  25                  30

Val Ala Ala Ala Leu Gly Arg Gly Pro Phe Pro Arg Val Pro Ala Val
        35                  40                  45

Leu Gly Trp Gln Gly Lys Gln Ala Asp Trp Lys Thr Arg Arg Trp Cys
    50                  55                  60

Ser Ser Gly Pro Val Pro Asn Glu Lys Ile Arg Asn Ile Gly Ile Ser
65                  70                  75                  80

Ala His Ile Asp Ser Gly Lys Thr Thr Leu Thr Glu Arg Val Leu Tyr
                85                  90                  95

Tyr Thr Gly Arg Ile Ala Thr Met His Glu Val Lys Gly Lys Asp Gly
            100                 105                 110

Val Gly Ala Val Met Asp Ser Met Glu Leu Glu Arg Gln Arg Gly Ile
        115                 120                 125

Thr Ile Gln Ser Ala Ala Thr Tyr Thr Met Trp Lys Asp Ile Asn Ile
    130                 135                 140

Asn Ile Ile Asp Thr Pro Gly His Val Asp Phe Thr Ile Glu Val Glu
145                 150                 155                 160

Arg Ala Leu Arg Val Leu Asp Gly Ala Val Leu Val Leu Cys Ala Val
                165                 170                 175

Gly Gly Val Gln Cys Gln Thr Met Thr Val Ser Arg Gln Met Lys Arg
            180                 185                 190

Tyr Asn Val Pro Phe Leu Thr Phe Ile Asn Lys Leu Asp Arg Met Gly
        195                 200                 205

Ser Asn Pro Ser Arg Ala Leu Gln Gln Met Arg Ser Lys Leu Asn His
    210                 215                 220

Asn Ala Ala Phe Val Gln Ile Pro Ile Gly Leu Glu Gly Asp Phe Lys
225                 230                 235                 240

Gly Ile Ile Asp Leu Ile Glu Glu Arg Ala Ile Tyr Phe Asp Gly Asp
                245                 250                 255

Phe Gly Gln Ile Val Arg Tyr Asp Glu Ile Pro Ala Gly Leu Arg Ala
            260                 265                 270

Ala Ala Ala Asp His Arg Gln Glu Leu Ile Glu Cys Val Ala Asn Ser
        275                 280                 285

Asp Glu Gln Leu Gly Glu Leu Phe Leu Glu Glu Lys Ile Pro Ser Val
    290                 295                 300

Ser Asp Leu Lys Arg Ala Ile Arg Arg Ala Thr Leu Ser Arg Ser Phe
305                 310                 315                 320

Thr Pro Val Phe Leu Gly Ser Ala Leu Lys Asn Lys Gly Val Gln Pro
                325                 330                 335

Leu Leu Asp Ala Val Leu Glu Tyr Leu Pro Asn Pro Ser Glu Val Gln
            340                 345                 350

Asn Tyr Ala Ile Leu Asn Gln Asn Asp Ser Lys Glu Lys Thr Lys Ile
        355                 360                 365

Leu Met Asn Pro Gln Arg Asp Asp Ser His Pro Phe Val Gly Leu Ala
    370                 375                 380

Phe Lys Leu Glu Ala Gly Arg Phe Gly Gln Leu Thr Tyr Val Arg Asn
385                 390                 395                 400

Tyr Gln Gly Glu Leu Lys Lys Gly Ser Thr Ile Tyr Asn Thr Arg Thr
                405                 410                 415

Gly Lys Lys Val Arg Val Gln Arg Leu Val Arg Met His Ala Asp Met
            420                 425                 430

Met Glu Asp Val Glu Glu Val Tyr Ala Gly Asp Ile Cys Ala Leu Phe
        435                 440                 445

Gly Ile Asp Cys Ala Ser Gly Asp Thr Phe Thr Asn Lys Asp Asn Ser
    450                 455                 460

Asp Leu Ser Met Glu Ser Ile His Val Pro Glu Pro Val Ile Ser Ile
465                 470                 475                 480

Ala Met Arg Pro Ser Asn Lys Asn Asp Leu Glu Lys Phe Ser Lys Gly
                485                 490                 495

Ile Gly Arg Phe Thr Arg Glu Asp Pro Thr Phe Lys Val His Phe Asp
            500                 505                 510

Pro Glu Ser Lys Glu Thr Ile Val Ser Gly Met Gly Glu Leu His Leu
        515                 520                 525

Glu Ile Tyr Ala Gln Arg Met Glu Arg Glu Tyr Gly Cys Pro Cys Ile
    530                 535                 540

Thr Gly Lys Pro Lys Val Ala Phe Arg Glu Thr Ile Val Ala Pro Val
545                 550                 555                 560

Pro Phe Asp Phe Thr His Lys Lys Gln Ser Gly Gly Ala Gly Gln Phe
                565                 570                 575

Gly Lys Val Ile Gly Val Leu Glu Pro Leu Pro Pro Glu Asp Tyr Thr
            580                 585                 590

Lys Leu Glu Phe Ser Asp Glu Thr Phe Gly Ser Asn Val Pro Lys Gln
        595                 600                 605

Phe Val Pro Ala Val Glu Lys Gly Phe Leu Asp Ala Cys Glu Lys Gly
    610                 615                 620

Pro Leu Ser Gly His Lys Leu Ser Gly Leu Arg Phe Val Leu Gln Asp
625                 630                 635                 640

Gly Ala His His Met Val Asp Ser Asn Glu Ile Ser Phe Ile Arg Ala
                645                 650                 655

Gly Glu Gly Ala Leu Lys Gln Ala Leu Ala Asn Gly Thr Leu Cys Ile
            660                 665                 670

Ile Glu Pro Ile Met Ser Val Glu Val Ile Ala Pro Asn Glu Phe Gln
        675                 680                 685

Gly Thr Val Phe Ala Gly Ile Asn Arg Arg His Gly Val Ile Thr Gly
    690                 695                 700

Gln Asp Gly Ile Glu Asp Tyr Phe Thr Leu Tyr Ala Asp Val Pro Leu
705                 710                 715                 720

Asn Asn Met Phe Gly Tyr Ser Thr Glu Leu Arg Ser Cys Thr Glu Gly
                725                 730                 735

Lys Gly Glu Tyr Thr Met Glu Tyr Cys Arg Tyr Gln Pro Cys Ser Pro
            740                 745                 750

Ser Thr Gln Glu Glu Leu Ile Asn Lys Tyr Leu Glu Ala Thr Gly Gln
        755                 760                 765

Leu Pro Val Lys Lys Gly Lys Ala Lys Asn Tyr Pro Tyr Asp Val Pro
    770                 775                 780

Asp Tyr Ala
785

<210>  23

<211>  34
<212>  DNA
<213>  Artificial

<220>
<223>  primer

<400>  23
agcatgacta gtatgagact cctgggagct gcag                                34


<210>  24
<211>  62
<212>  DNA
<213>  Artificial

<220>
<223>  primer

<400>  24
tcatgcctcg agttaagcgt aatctggtac gtcgtatggg tagttcttgg cttttccttt      60

tt                                                                   62


<210>  25
<211>  787
<212>  PRT
<213>  Artificial

<220>
<223>  fusion protein

<400>  25

His His His His His His Gly Met Gly Ala Ala Gly Arg Lys Lys Arg
1               5                   10                  15


Arg Gln Arg Arg Arg Pro Pro Ala Gly Thr Ser Met Arg Leu Leu Gly
            20                  25                  30


Ala Ala Ala Val Ala Ala Leu Gly Arg Gly Arg Ala Pro Ala Ser Leu
        35                  40                  45


Gly Trp Gln Arg Lys Gln Val Asn Trp Lys Ala Cys Arg Trp Ser Ser
    50                  55                  60


Ser Gly Val Ile Pro Asn Glu Lys Ile Arg Asn Ile Gly Ile Ser Ala
65                  70                  75                  80


His Ile Asp Ser Gly Lys Thr Thr Leu Thr Glu Arg Val Leu Tyr Tyr
                85                  90                  95


Thr Gly Arg Ile Ala Lys Met His Glu Val Lys Gly Lys Asp Gly Val
            100                 105                 110

Gly Ala Val Met Asp Ser Met Glu Leu Glu Arg Gln Arg Gly Ile Thr
        115                 120                 125

Ile Gln Ser Ala Ala Thr Tyr Thr Met Trp Lys Asp Val Asn Ile Asn
    130                 135                 140

Ile Ile Asp Thr Pro Gly His Val Asp Phe Thr Ile Glu Val Glu Arg
145                 150                 155                 160

Ala Leu Arg Val Leu Asp Gly Ala Val Leu Val Leu Cys Ala Val Gly
                165                 170                 175

Gly Val Gln Cys Gln Thr Met Thr Val Asn Arg Gln Met Lys Arg Tyr
            180                 185                 190

Asn Val Pro Phe Leu Thr Phe Ile Asn Lys Leu Asp Arg Met Gly Ser
        195                 200                 205

Asn Pro Ala Arg Ala Leu Gln Gln Met Arg Ser Lys Leu Asn His Asn
    210                 215                 220

Ala Ala Phe Met Gln Ile Pro Met Gly Leu Glu Gly Asn Phe Lys Gly
225                 230                 235                 240

Ile Val Asp Leu Ile Glu Glu Arg Ala Ile Tyr Phe Asp Gly Asp Phe
                245                 250                 255

Gly Gln Ile Val Arg Tyr Gly Glu Ile Pro Ala Glu Leu Arg Ala Ala
            260                 265                 270

Ala Thr Asp His Arg Gln Glu Leu Ile Glu Cys Val Ala Asn Ser Asp
        275                 280                 285

Glu Gln Leu Gly Glu Met Phe Leu Glu Glu Lys Ile Pro Ser Ile Ser
    290                 295                 300

Asp Leu Lys Leu Ala Ile Arg Arg Ala Thr Leu Lys Arg Ser Phe Thr
305                 310                 315                 320

Pro Val Phe Leu Gly Ser Ala Leu Lys Asn Lys Gly Val Gln Pro Leu
                325                 330                 335

Leu Asp Ala Val Leu Glu Tyr Leu Pro Asn Pro Ser Glu Val Gln Asn
            340                 345                 350

Tyr Ala Ile Leu Asn Lys Glu Asp Asp Ser Lys Glu Lys Thr Lys Ile
        355                 360                 365

Leu Met Asn Ser Ser Arg Asp Asn Ser His Pro Phe Val Gly Leu Ala
    370                 375                 380

Phe Lys Leu Glu Val Gly Arg Phe Gly Gln Leu Thr Tyr Val Arg Ser
385                 390                 395                 400

Tyr Gln Gly Glu Leu Lys Lys Gly Asp Thr Ile Tyr Asn Thr Arg Thr
                405                 410                 415

Arg Lys Lys Val Arg Leu Gln Arg Leu Ala Arg Met His Ala Asp Met
            420                 425                 430

Met Glu Asp Val Glu Glu Val Tyr Ala Gly Asp Ile Cys Ala Leu Phe
        435                 440                 445

Gly Ile Asp Cys Ala Ser Gly Asp Thr Phe Thr Asp Lys Ala Asn Ser
    450                 455                 460

Gly Leu Ser Met Glu Ser Ile His Val Pro Asp Pro Val Ile Ser Ile
465                 470                 475                 480

Ala Met Lys Pro Ser Asn Lys Asn Asp Leu Glu Lys Phe Ser Lys Gly
                485                 490                 495

Ile Gly Arg Phe Thr Arg Glu Asp Pro Thr Phe Lys Val Tyr Phe Asp
            500                 505                 510

Thr Glu Asn Lys Glu Thr Val Ile Ser Gly Met Gly Glu Leu His Leu
        515                 520                 525

Glu Ile Tyr Ala Gln Arg Leu Glu Arg Glu Tyr Gly Cys Pro Cys Ile
    530                 535                 540

Thr Gly Lys Pro Lys Val Ala Phe Arg Glu Thr Ile Thr Ala Pro Val
545                 550                 555                 560

Pro Phe Asp Phe Thr His Lys Lys Gln Ser Gly Gly Ala Gly Gln Tyr
                565                 570                 575

Gly Lys Val Ile Gly Val Leu Glu Pro Leu Asp Pro Glu Asp Tyr Thr
            580                 585                 590

Lys Leu Glu Phe Ser Asp Glu Thr Phe Gly Ser Asn Ile Pro Lys Gln
        595                 600                 605

Phe Val Pro Ala Val Glu Lys Gly Phe Leu Asp Ala Cys Glu Lys Gly
    610                 615                 620

Pro Leu Ser Gly His Lys Leu Ser Gly Leu Arg Phe Val Leu Gln Asp
625                 630                 635                 640

Gly Ala His His Met Val Asp Ser Asn Glu Ile Ser Phe Ile Arg Ala
                645                 650                 655

Gly Glu Gly Ala Leu Lys Gln Ala Leu Ala Asn Ala Thr Leu Cys Ile
            660                 665                 670

Leu Glu Pro Ile Met Ala Val Glu Val Val Ala Pro Asn Glu Phe Gln
        675                 680                 685

Gly Gln Val Ile Ala Gly Ile Asn Arg Arg His Gly Val Ile Thr Gly
    690                 695                 700

Gln Asp Gly Val Glu Asp Tyr Phe Thr Leu Tyr Ala Asp Val Pro Leu
705                 710                 715                 720

Asn Asp Met Phe Gly Tyr Ser Thr Glu Leu Arg Ser Cys Thr Glu Gly
                725                 730                 735

Lys Gly Glu Tyr Thr Met Glu Tyr Ser Arg Tyr Gln Pro Cys Leu Pro
            740                 745                 750

Ser Thr Gln Glu Asp Val Ile Asn Lys Tyr Leu Glu Ala Thr Gly Gln
        755                 760                 765

Leu Pro Val Lys Lys Gly Lys Ala Lys Asn Tyr Pro Tyr Asp Val Pro
    770                 775                 780

Asp Tyr Ala
785

<210>  26
<211>  16
<212>  PRT
<213>  Artificial

<220>
<223>  protein transduction domain

<400>  26

Arg Gln Ile Lys Ile Trp Phe Gln Asn Arg Arg Met Lys Trp Lys Lys
1               5                   10                  15

<210>  27
<211>  16
<212>  PRT

<213>  Artificial

<220>
<223>  protein transduction domain

<400>  27

Arg Gln Ile Leu Ile Trp Phe Gln Asn Arg Arg Met Lys Trp Leu Leu
1               5                   10                  15


<210>  28
<211>  11
<212>  PRT
<213>  Artificial

<220>
<223>  protein transduction domain

<400>  28

Tyr Ala Arg Ala Ala Ala Arg Gln Ala Arg Ala
1               5                   10


<210>  29
<211>  12
<212>  PRT
<213>  Artificial

<220>
<223>  protein transduction domain

<400>  29

Pro Ile Arg Arg Arg Lys Lys Leu Arg Arg Leu Lys
1               5                   10


<210>  30
<211>  12
<212>  PRT
<213>  Artificial

<220>
<223>  protein transduction domain

<400>  30

Arg Arg Gln Arg Arg Thr Ser Lys Leu Met Lys Arg
1               5                   10


<210>  31
<211>  18
<212>  PRT
<213>  Artificial

<220>
<223>  protein transduction domain

<400>  31

Arg Gly Gly Arg Leu Ser Tyr Ser Arg Arg Arg Phe Ser Thr Ser Thr
1               5                   10                  15

Gly Arg

<210>  32
<211>  10
<212>  PRT
<213>  Artificial

<220>
<223>  protein transduction domain

<400>  32

Arg Arg Leu Ser Tyr Ser Arg Arg Arg Phe
1               5                   10

<210>  33
<211>  15
<212>  PRT
<213>  Artificial

<220>
<223>  protein transduction domain

<400>  33

Arg Arg Arg Arg Asn Arg Thr Arg Arg Asn Arg Arg Arg Val Arg
1               5                   10                  15

<210>  34
<211>  19
<212>  PRT
<213>  Artificial

<220>
<223>  protein transduction domain

<400>  34

Lys Met Thr Arg Ala Gln Arg Arg Ala Ala Ala Arg Arg Asn Arg Trp
1               5                   10                  15

Thr Ala Arg

<210>  35
<211>  13
<212>  PRT
<213>  Artificial

<220>
<223>  protein transduction domain

<400>  35

Thr Arg Arg Gln Arg Thr Arg Arg Ala Arg Arg Asn Arg
1               5                   10


<210>  36
<211>  13
<212>  PRT
<213>  Artificial

<220>
<223>  protein transduction domain

<400>  36

Gly Arg Lys Lys Arg Arg Gln Arg Arg Arg Pro Pro Gln
1               5                   10


<210>  37
<211>  13
<212>  PRT
<213>  Artificial

<220>
<223>  protein transduction domain

<400>  37

Gly Arg Arg Arg Arg Arg Arg Arg Arg Arg Pro Pro Gln
1               5                   10


<210>  38
<211>  34
<212>  PRT
<213>  Artificial

<220>
<223>  protein transduction domain

<400>  38

Asp Ala Ala Thr Ala Thr Arg Gly Arg Ser Ala Ala Ser Arg Pro Thr
1               5                   10                  15


Glu Arg Pro Arg Ala Pro Ala Arg Ser Ala Ser Arg Pro Arg Arg Pro
            20                  25                  30


Val Glu



<210>  39
<211>  15
<212>  PRT
<213>  Artificial

<220>
<223>  protein transduction domain

<400>  39

Gln Ala Arg Arg Asn Arg Arg Arg Arg Trp Arg Glu Arg Gln Arg
1               5                   10                  15


<210>  40
<211>  19
<212>  PRT
<213>  Artificial

<220>
<223>  protein transduction domain

<400>  40

Met Pro Lys Thr Arg Arg Arg Pro Arg Arg Ser Gln Arg Lys Arg Pro
1               5                   10                  15


Pro Thr Pro




<210>  41
<211>  27
<212>  PRT
<213>  Artificial

<220>
<223>  protein transduction domain

<400>  41

Gly Trp Thr Leu Asn Ser Ala Gly Tyr Leu Leu Gly Lys Ile Asn Leu
1               5                   10                  15


Lys Ala Leu Ala Ala Leu Ala Lys Lys Ile Leu
            20                  25


<210>  42
<211>  17
<212>  PRT
<213>  Artificial

<220>
<223>  protein transduction domain

<400>  42

Lys Leu Ala Leu Lys Leu Ala Leu Lys Leu Ala Leu Ala Leu Lys Leu
1               5                   10                  15


Ala




<210>  43
<211>  27
<212>  PRT

<213>  Artificial

<220>
<223>  protein transduction domain

<400>  43

Met Gly Leu Gly Leu His Leu Leu Val Leu Ala Ala Ala Leu Gln Gly
1               5                   10                  15

Ala Trp Ser Gln Pro Lys Lys Lys Arg Lys Val
            20                  25

<210>  44
<211>  27
<212>  PRT
<213>  Artificial

<220>
<223>  protein transduction domain

<400>  44

Gly Ala Leu Phe Leu Gly Trp Leu Gly Ala Ala Gly Ser Thr Met Gly
1               5                   10                  15

Ala Trp Ser Gln Pro Lys Lys Lys Arg Lys Val
            20                  25

<210>  45
<211>  27
<212>  PRT
<213>  Artificial

<220>
<223>  protein transduction domain

<400>  45

Gly Ala Leu Phe Leu Gly Phe Leu Gly Ala Ala Gly Ser Thr Met Gly
1               5                   10                  15

Ala Trp Ser Gln Pro Lys Lys Lys Arg Lys Val
            20                  25

<210>  46
<211>  27
<212>  PRT
<213>  Artificial

<220>
<223>  protein transduction domain

<400>  46

Gly Ala Leu Phe Leu Gly Phe Leu Gly Ala Ala Gly Ser Thr Met Gly
1               5                   10                  15

Ala Trp Ser Gln Pro Lys Ser Lys Arg Lys Val
            20                  25

<210>  47
<211>  21
<212>  PRT
<213>  Artificial

<220>
<223>  protein transduction domain

<400>  47

Lys Glu Thr Trp Trp Glu Thr Trp Trp Thr Glu Trp Ser Gln Pro Lys
1               5                   10                  15

Lys Lys Arg Lys Val
            20

<210>  48
<211>  21
<212>  PRT
<213>  Artificial

<220>
<223>  protein transduction domain

<400>  48

Lys Glu Thr Trp Phe Glu Thr Trp Phe Thr Glu Trp Ser Gln Pro Lys
1               5                   10                  15

Lys Lys Arg Lys Val
            20

## Description

The present invention relates to the field of treatment of mitochondrial diseases.

Mitochondria generate the majority of cellular adenosine triphosphate (ATP) by the mitochondrial oxidative phosphorylation (OXPHOS). The OXPHOS system comprises five multisubunit enzyme complexes located in the inner mitochondrial membrane. Mitochondria are semi-autonomous organelles: They contain their own genome (mtDNA), but rely on the nuclear DNA for full functionality. The nuclear genome encodes about 1500 mitochondrial proteins and provides essential factors for mtDNA replication, transcription, translation and assembly of the five OXPHOS complexes. The mitochondrial DNA is a minimal genome and only encodes for 13 essential subunits of the OXPHOS system as well as 2 rRNAs and 22 tRNAs. Except for complex II, all OXPHOS enzymes derive from dual genetic origin with at least one subunit encoded by the mtDNA making mitochondrial protein synthesis crucial for OXPHOS function and ATP generation.

Both nuclear-encoded and mitochondrial-encoded proteins can be mutated and give rise to mitochondrial dysfunction. Genetic alterations affecting mitochondrial proteins can cause severe and often fatal disorders. To date, treatment of diseases involving mitochondrial proteins still is restricted to symptom management and lack of established causative therapies. Document WO 2012/174452 A1 for example discloses a targeted therapeutic comprising a mitochondrial targeting sequence to target frataxin to mitochondria for treatment of Friedreich's Ataxia. Although Friedreich's Ataxia patients suffer dysfunction in mitochondria, frataxin, however, is not associated to the mitochondrial oxidative phosphorylation system.

Genetic alterations affecting the mitochondrial oxidative phosphorylation system give rise to mitochondrial disorders, a heterogenous group of diseases with a bioenergetic deficiency. Due to the compromised ATP supply, high energy-demand tissues like skeletal muscle, heart, liver and brain are most severely affected in mitochondrial diseases. Genetic alterations affecting this system give rise to particularly severe and often fatal mitochondrial disorders. In spite of the progress made during the last two decades in deciphering the molecular mechanisms and advancing the diagnostics of mitochondrial diseases, major improvements in treatment are still lacking. Hereto adds that mitochondrial disease in humans can present at any age, and practically in any organ system. Particularly a high incidence of mid- and late pregnancy loss is a common occurrence that often goes unrecognized.

Therefore, the object underlying the present invention was to provide a means being usable in the treatment of mitochondrial diseases.

The problem is solved by a fusion protein comprising:
a) a protein transduction domain,
b) a mitochondrial targeting sequence, and
c) a mitochondrial elongation factor G1 peptide.

It was found that, surprisingly, the fusion protein comprising a mitochondrial elongation factor G1 (EF-G1) peptide effectively enriched in mitochondria when living cells were transduced and could complement EF-G1 deficiency and the resulting oxidative phosphorylation (OXPHOS) defect in the cells while they were proliferating. It was particularly advantageously found that treatment with the EF-G1 fusion protein partially restored mitochondrial OXPHOS proteins in the brain of EF-G1 knock-out mice, whereas no negative effect in wild type animals and non-affected tissues was observed. These findings indicate that the EF-G1 fusion protein penetrates the blood-brain barrier and partially relieves the protein deficiency and ameliorates the OXPHOS defect.

As used herein, the term "peptide" refers to a chain of amino acids linked to one another by a peptide bond. A peptide may include a short chain of at least 2 to 5 amino acids, in other embodiments a peptide may include a long, continuous peptide chain which also may be referred to as a polypeptide or protein. If not stated otherwise, the term peptide may be used synonym to polypeptide or protein.

As used herein, the term "fusion protein" refers to a sequence of amino acids, wherein a part of the sequence is derived from one origin and fused to another part of the sequence that is derived from one or more other origin. The origin of a protein or an amino acid sequence may be native or synthetic, such as for a tag or linker. This term refers to the original source of the sequences, as in practice when the fusion protein is prepared by recombinant techniques there is no distinction between the fused parts.

The term "protein transduction domain" as used herein refers to an amino acid sequence capable of enabling the transport of a peptide or sequence attached to it through the cellular membranes. It is assumed that transport by a protein transduction domain is independent of receptor-mediated entry or phagocytosis. A protein transduction domain particularly enables a transport through the cell membrane. Such protein transduction domains are particularly useful for the present invention. Suitable protein transduction domains are cationic peptides that are heavily positively charged by being rich in positive amino acids such as arginine or lysine such as a HIV-I Trans Activator of Transcription (TAT) sequence. Suitable protein transduction domains can be produced using recombinant and synthetic methods or purified from natural sources. Hence, a protein transduction domain may encompass a naturally occurring sequence or a modified sequence that retains cell-penetrating properties.

The term "mitochondrial targeting sequence" (MTS) as used herein refers to an amino acid sequence capable of directing the transport of a peptide or sequence attached to it into the mitochondria. Suitable mitochondrial targeting sequences include sequences that mediate mitochondrial localization of the fused protein and provide a moiety for cleavage of the MTS within the mitochondrial compartment but not within the cellular cytoplasm of a target cell into which the peptide or protein is to be delivered. The MTS may be a human MTS, or may be an MTS from another species. Suitable mitochondrial targeting sequences can be produced using recombinant and synthetic methods or purified from natural sources. Hence, a mitochondrial targeting sequence may encompass a naturally-occurring sequence or a modified sequence that retains mitochondrial targeting properties.

A fusion protein containing a protein transduction domain, a mitochondrial targeting sequence and a protein can transduce into a cell and into a mitochondria. If a fusion protein containing a MTS transduces into mitochondria, the mitochondrial processing peptidase will recognize the MTS and clip it, so that the mature mitochondrial elongation factor G1 peptide can reside in the mitochondrial matrix.

As used herein, the term "mitochondrial elongation factor G1" or "EF-G1" refers to a mitochondrial protein, which is also referred to as Gfm1 or GFM1. Although GFM1 (G elongation factor, mitochondrial 1) in the proper meaning refers to the protein-coding gene while EF-G1 refers to the protein, EF-G1 and GFM1 are widely used synonymous to refer to the mitochondrial elongation factor G1 protein. As used herein, the terms "mitochondrial elongation factor G1", "EF-G1" and Gmf1 or GFM1 are used synonymously. Further, the terms "EF-G1 fusion protein" and "GFM1 fusion protein" herein are used synonymously. The function attributed to the mitochondrial elongation factor G1 is that of a mitochondrial GTPase that catalyzes the GTP-dependent ribosomal translocation step during translation elongation. A representative amino acid sequence of the full length human Gfm1 precursor protein is a consecutive order of SEQ ID NO: 1 and SEQ ID NO: 2 resulting in a 751 amino acid protein as set forth in GenBank Accession No. NP_079272.4.

The mitochondrial elongation factor G1 peptide can be a protein fragment sufficient to carry out the enzymatic activity of the elongation factor G1. The term "mitochondrial elongation factor G1 peptide" as used herein refers to any fragment of the enzyme comprising the catalytic domain thereof, wherein the conformation of the fragment under physiological conditions is such that the enzymatic activity is maintained. Preferably, the mitochondrial elongation factor G1 peptide refers to the enzymatically active EF-G1 protein. A preferred mitochondrial elongation factor G1 protein refers to the C-terminal part of Gfm1 without its natural MTS. The protein sequence advantageously provides the mature form of the protein providing the full enzymatic activity of the natural protein. The mitochondrial elongation factor G1 preferably is mammalian in origin. Most preferably, the mitochondrial elongation factor G1 is human in origin. The mitochondrial elongation factor G1 may also be of mouse or other mammalian origin.

In an embodiment, the mitochondrial elongation factor G1 peptide comprises the following sequence SGVIPNEKIRNIGISAHIDSGKTTLTERVLYYTGRIAKMHEVKGKDGVGAVMDSMELERQRGI TIQSAATYTMWKDVNINIIDTPGHVDFTIEVERALRVLDGAVLVLCAVGGVQCQTMTVNRQ MKRYNVPFLTFINKLDRMGSNPARALQQMRSKLNHNAAFMQIPMGLEGNFKGIVDLIEERAI YFDGDFGQIVRYGEIPAELRAAATDHRQELIECVANSDEQLGEMFLEEKIPSISDLKLAIRRATL KRSFTPVFLGSALKNKGVQPLLDAVLEYLPNPSEVQNYAILNKEDDSKEKTKILMNSSRDNSH PFVGLAFKLEVGRFGQLTYVRSYQGELKKGDTIYNTRTRKKVRLQRLARMHADMMEDVEE VYAGDICALFGIDCASGDTFTDKANSGLSMESIHVPDPVISIAMKPSNKNDLEKFSKGIGRFTR EDPTFKVYFDTENKETVISGMGELHLEIYAQRLEREYGCPCITGKPKVAFRETITAPVPFDFTH KKQSGGAGQYGKVIGVLEPLDPEDYTKLEFSDETFGSNIPKQFVPAVEKGFLDACEKGPLSGH KLSGLRFVLQDGAHHMVDSNEISFIRAGEGALKQALANATLCILEPIMAVEWAPNEFQGQVI AGINRRHGVITGQDGVEDYFTLYADVPLNDMFGYSTELRSCTEGKGEYTMEYSRYQPCLPST QEDVINKYLEATGQLPVKKGKAKN (SEQ ID NO: 2). The SEQ ID NO: 2 refers to the enzymatic active part of human Gfm1, corresponding to the amino acids 38 to 751 of the 751 amino acid protein set forth in GenBank Accession No. NP_079272.4.In embodiments, the mitochondrial elongation factor G1 peptide without MTS and protein transduction domain also is referred to as mature Gfm1 protein.

In other embodiments, the mitochondrial elongation factor G1 peptide may be a mutated derivative wherein one or more of the native amino acids is deleted, replaced or modified while the derivative still maintains the enzymatic functionally of the protein or at least 30 % of the enzymatic functionality of the protein. In embodiments, the mitochondrial elongation factor G1 peptide has at least 80% sequence identity to SEQ ID NO: 2. In further embodiments, the mitochondrial elongation factor G1 peptide has at least 90% or 95% sequence identity to SEQ ID NO: 2.

In the sequence of a peptide certain amino acids can be substituted for other amino acids, as long a protein still maintains its enzymatic activity or at least 30 % of its enzymatic activity. Preferred changes are referred to as conservative amino acid substitutions. A conservative substitution replaces one amino acid with another that is similar in size and/or chemical properties. Such conservative amino acid substitutions may have minor effects on protein structure and can thus be tolerated without compromising function. Conservative amino acid substitutions may substitute amino acids that are typically classified as being hydrophobic or hydrophilic, or as having polar or non-polar, acidic or basic side chains for another of the same type. The term "identity" refers to a comparison between amino acid sequences. Identity or similarity can be determined as a degree of sequence relatedness between sequences. Sequence identity can be calculated by known methods using a variety of algorithms. Amino acid sequences for example may be compared using available commercial computer programs such as BLASTP.

The fusion protein further comprises a mitochondria targeting sequence (MTS). An MTS can be the natural MTS of the elongation factor G1 or be an MTS of another mitochondrial protein that is in the original context to be transported into the mitochondria. A mitochondrial targeting sequence may be derived from mitochondrial proteins destined for the mitochondrial matrix. Examples for suitable mitochondria targeting sequences may be the mitochondrial malate dehydrogenase cleavage sequence or the mitochondrial creatine kinase (MtCK) sequence. Other examples may be the ornithine transcarbamylase targeting sequence, or a targeting sequence from a nuclear-encoded subunit of the NADH complex.

In embodiments, the mitochondrial targeting sequence is selected from the group comprising MRLLGAAAVAALGRGRAPASLGWQRKQVNWKACRWSS (SEQ ID NO: 1), MSVLTPLLLRGLTGSARRLPVPRAK (SEQ ID NO: 3), MLSRAVCGTSRQLAPVLGYLGSRQ (SEQ ID NO: 4) and/or MAFLRSMWGVLSALGRSGA (SEQ ID NO: 5), or has at least 80% sequence identity to SEQ ID NOs: 1, or 3 to 5. In further preferred embodiments, the mitochondrial targeting sequence has at least 90% or 95% sequence identity to SEQ ID NOs: 1, or 3 to 5.

In a preferred embodiment, the MTS has the SEQ ID NO: 1. SEQ ID NO: 1 represents the native mitochondrial targeting sequence found in mitochondrial elongation factor G1. SEQ ID NO: 1 corresponds to the amino acids of positions 1 to 37 of human Gfm1 as set forth in GenBank Accession No. NP_079272.4.Preferably, the precursor protein of mitochondrial elongation factor G1 including its natural MTS and enzymatic active part is used. In a preferred embodiment, the fusion protein comprises the MTS of SEQ ID NO: 1 and the peptide of SEQ ID NO: 2 of human Gfm1. Thus, the entire wild-type sequence of the enzyme may be used. In this precursor protein the MTS is directly fused to the EF-G1 peptide. In this embodiment, cleavage of the MTS generates a mature elongation factor G1 with the native sequence. Using the precursor mitochondrial elongation factor G1 provides for an advantageous compatibility if used with its natural subject, and a protein able to fulfill is destined enzymatic action.

However, the sequence alternatively may be one that is not the MTS naturally associated with EF-G1. Preferred heterologous targeting sequences are selected from the group of SEQ ID NOs: 3 to 5. SEQ ID NOs: 3, 4 and 5 represent the targeting sequences of mitochondrial proteins cytochrome c oxidase subunit VIII (COX8), superoxide dismutase 2 (SOD2) and transcription factor A, mitochondrial (TFAM), respectively. An exchange of the MTS may be advantageously to increase translocation efficacy or support bacterial expression and protein purification.

Usually, the mitochondrial targeting sequence is located at the amino terminus of a mitochondrial precursor protein. In embodiments, the mitochondrial targeting sequence links the protein transduction domain and the EF-G1 protein or peptide.

Suitable protein transduction domains having cell-penetrating properties may be a hydrophilic or amphiphilic sequence. A suitable hydrophilic sequence may be selected from the group comprising RQIKIWFQNRRMKWKK (Antennapedia, SEQ ID NO:26), RQILIWFQNRRMKWLL (Antennapedia, SEQ ID NO:27), YARAAARQARA (PTD-4, SEQ ID NO:28), PIRRRKKLRRLK (PTD-4, SEQ ID NO:29), RRQRRTSKLMKR (PTD-5, SEQ ID NO:30), RGGRLSYSRRRFSTSTGR (SynB1, SEQ ID NO:31), RRLSYSRRRF (SynB3, SEQ ID NO:32), RRRRNRTRRNRRRVR (FHV Coat-(35-49), SEQ ID NO:33), KMTRAQRRAAARRNRWTAR (BMV Gag-(7-25), SEQ ID NO:34), TRRQRTRRARRNR (HTLV-II Rex-(4-16), SEQ ID NO:35), GRKKRRQRRRPPQ (D-Tat, SEQ ID NO:36), GRRRRRRRRRPPQ (R9-Tat, SEQ ID NO:37), DAATATRGRSAASRPTERPRAPARSASRPRRPVE (VP22, SEQ ID NO:38), QARRNRRRRWRERQR-51 (HIV-1 Rev protein basic motif, SEQ ID NO:39) and/or MPKTRRRPRRSQRKRPPTP-119 (HTLV-1 Rex protein basic motif, SEQ ID NO:40). A suitable amphiphilic sequence may be selected from the group comprising GWTLNSAGYLLGKINLKALAALAKKIL (Transportan chimera, SEQ ID NO:41), KLALKLALKLALALKLA (MAP, SEQ ID NO:42), MGLGLHLLVLAAALQGAWSQPKKKRKV (SBP, SEQ ID NO:43), GALFLGWLGAAGSTMGAWSQPKKKRKV (FBP, SEQ ID NO:44), ac-GALFLGFLGAAGSTMGAWSQPKKKRKV-cya (MPG, SEQ ID NO:45), ac-GALFLGFLGAAGSTMGAWSQPKSKRKV-cya (MPG(ΔNLS), SEQ ID NO:46), ac-KETWWETWWTEWSQPKKKRKV-cya (Pep-1, SEQ ID NO:47) and/or ac-KETWFETWFTEWSQPKKKRKV-cya (Pep-2, SEQ ID NO:48). In embodiments, the protein transduction domain may be derived from potential DNA binding proteins such as HIV-TAT, VP22, and Antennapedia.

A preferred protein transduction domain is a HIV-I Trans Activator of Transcription (TAT) peptide. TAT peptides are capable of traversing the cellular and mitochondrial membranes of eukaryotic cells. A TAT sequence conjugated to a mitochondrial protein that does not normally cross cellular membranes is able to deliver a biologically active fusion protein to tissues by transducing the fusion protein across the cell membranes. Advantageously, TAT-fusion proteins can transduce cultured cells, intact tissue, and live tissues. Most advantageously, TAT-fusion proteins penetrate the blood-brain barrier to transduce brain matter.

In embodiments, the protein transduction domain is a TAT sequence selected from the group comprising YGRKKRRQRRR (SEQ ID NO: 6), GRKKRRQRRR (SEQ ID NO: 7) and/or GRKKRRQRRRPQ (SEQ ID NO: 8), or has at least 80% sequence identity to SEQ ID NOs: 6 to 8. In further preferred embodiments, the TAT sequence has at least 90% or 95% sequence identity to SEQ ID NOs: 6 to 8. Advantageously, TAT sequences are well tolerated in cells and tissue. No or only minimal detrimental effects particularly no neurological problems or system distress is expected of TAT induced protein transduction.

The protein transduction domain may be fused to the mitochondrial targeting sequence via a linker. As used herein, the term "linker" refers to an amino acid sequence that is not naturally provided at a particular position in the natural protein. A linker may function as a spacer or provide a certain degree of flexibility between two peptide moieties. Suitable linkers may be selected from the group comprising PPAGTS (SEQ ID NO: 17) and GMGGA (SEQ ID NO: 18). In embodiments, the TAT sequence may be fused to the MTS via the linker of SEQ ID NO: 17.

The fusion protein may be produced by recombinant techniques. Suitable host expression systems include bacteria, egg, baculovirus, plant, yeast, or mammalian cells. In embodiments, fusion proteins are produced in bacterial cells. Bacterial cells that may be used are E. coli. Fusion proteins also may be produced in mammalian cells.

A suitable fusion protein or vector may be constructed to encode a tag. A tag may advantageously be fused to a fusion protein to facilitate purification from the biological expression system using affinity technique or to allow for a detection of the fusion protein in a certain cell after protein transduction. In embodiments, the fusion protein comprises at least one N-terminal and/or C-terminal tag selected from the group comprising HHHHHH (SEQ ID NO: 9), HHHHHHHHHH (SEQ ID NO: 10), AWRHPQFGG (SEQ ID NO: 11), WSHPQFEK (SEQ ID NO: 12), YPYDVPDYA (SEQ ID NO: 13), EQKLISEEDL (SEQ ID NO: 14) and/or DYKDDDDK (SEQ ID NO: 15), or a tag having at least 80% sequence identity to SEQ ID NOs: 9 to 15. In further preferred embodiments, the tag has at least 90% or 95% sequence identity to SEQ ID NOs: 9 to 15.

Particularly poly HIS tags such as of SEQ ID NOs: 9 or 10 or GST tags can N-terminally be fused to the fusion protein to facilitate purification. HIS tags bind to metal matrices. An N-terminal HIS tag may be fused to the TAT sequence of the fusion protein via a linker such as the linker of SEQ ID NO: 18. The human influenza hemagglutinin (HA) is used as a general epitope tag in expression vectors. In embodiments, the HA tag of SEQ ID NO: 13 is fused C-terminally to the fusion protein. Advantageously, the HA tag does not appear to interfere with the bioactivity or the biodistribution of the fusion protein and allows for a detection after a protein transduction into cells or living tissue. The C-terminal HA tag may be directly fused to the C-terminal end of the EF-G1 peptide.

In embodiments, the fusion protein comprises a protein transduction domain being a TAT sequence, and the natural mitochondrial targeting sequence of the human mitochondrial elongation factor G1 peptide. In preferred embodiments, the fusion protein comprises a TAT sequence and the full-length human EF-G1 precursor protein including the MTS. In a preferred embodiment, the fusion protein has the following sequence HHHHHHGMGAAGRKKRRQRRRPPAGTSMRLLGAAAVAALGRGRAPASLGWQRKQVNWK ACRWSSSGVIPNEKIRNIGISAHIDSGKTTLTERVLYYTGRIAKMHEVKGKDGVGAVMDSMEL ERQRGITIQSAATYTMWKDVNINIIDTPGHVDFTIEVERALRVLDGAVLVLCAVGGVQCQTM TVNRQMKRYNVPFLTFINKLDRMGSNPARALQQMRSKLNHNAAFMQIPMGLEGNFKGIVDL IEERAIYFDGDFGQIVRYGEIPAELRAAATDHRQELIECVANSDEQLGEMFLEEKIPSISDLKLAI RRATLKRSFTPVFLGSALKNKGVQPLLDAVLEYLPNPSEVQNYAILNKEDDSKEKTKILMNSS RDNSHPFVGLAFKLEVGRFGQLTYVRSYQGELKKGDTIYNTRTRKKVRLQRLARMHADMM EDVEEVYAGDICALFGIDCASGDTFTDKANSGLSMESIHVPDPVISIAMKPSNKNDLEKFSKGI GRFTREDPTFKVYFDTENKETVISGMGELHLEIYAQRLEREYGCPCITGKPKVAFRETITAPVP FDFTHKKQSGGAGQYGKVIGVLEPLDPEDYTKLEFSDETFGSNIPKQFVPAVEKGFLDACEKG PLSGHKLSGLRFVLQDGAHHMVDSNEISFIRAGEGALKQALANATLCILEPIMAVEVVAPNEF QGQVIAGINRRHGVITGQDGVEDYFTLYADVPLNDMFGYSTELRSCTEGKGEYTMEYSRYQP CLPSTQEDVINKYLEATGQLPVKKGKAKN (SEQ ID NO: 16), or has at least 80% sequence identity to SEQ ID NO: 16. In further embodiments, the fusion protein has at least 90% or 95% sequence identity to SEQ ID NO: 16. The fusion protein of SEQ ID NO: 16 comprises N-terminally the HIS tag of SEQ ID NO: 9 that is fused via the linker of SEQ ID NO: 18 to the TAT sequence of SEQ ID NO: 7, followed by the linker of SEQ ID NO: 17 fusing the TAT sequence to the natural precursor protein of human EF-G1 which comprises its natural mitochondrial targeting sequence of SEQ ID NO: 1 and the enzymatic active protein of SEQ ID NO: 2. In embodiments, the fusion protein further comprises C-terminally the HA tag of SEQ ID NO: 13. The fusion protein of SEQ ID NO: 16 comprising human EF-G1 is particularly of therapeutic relevance.

In other embodiments, the fusion protein comprises a protein transduction domain being a TAT sequence, and the natural mitochondrial targeting sequence of the mouse mitochondrial elongation factor G1 peptide. In an embodiment, the fusion protein has the following sequence HHHHHHGMGAAGRKKRRQRRRPPAGTSMRLLRVAAALGRGPFPRVPAVLGWQGKQADWK TRRWCSSGPVPNEKIRNIGISAHIDSGKTTLTERVLYYTGRIATMHEVKGKDGVGAVMDSME LERQRGITIQSAATYTMWKDININIIDTPGHVDFTIEVERALRVLDGAVLVLCAVGGVQCQTM TVSRQMKRYNVPFLTFINKLDRMGSNPSRALQQMRSKLNHNAAFVQIPIGLEGDFKGIIDLIEE RAIYFDGDFGQIVRYDEIPAGLRAAAADHRQELIECVANSDEQLGELFLEEKIPSVSDLKRAIR RATLSRSFTPVFLGSALKNKGVQPLLDAVLEYLPNPSEVQNYAILNQNDSKEKTKILMNPQRD DSHPFVGLAFKLEAGRFGQLTYVRNYQGELKKGSTIYNTRTGKKVRVQRLVRMHADMMED VEEVYAGDICALFGIDCASGDTFTNKDNSDLSMESIHVPEPVISIAMRPSNKNDLEKFSKGIGR FTREDPTFKVHFDPESKETIVSGMGELHLEIYAQRMEREYGCPCITGKPKVAFRETIVAPVPFD FTHKKQSGGAGQFGKVIGVLEPLPPEDYTKLEFSDETFGSNVPKQFVPAVEKGFLDACEKGPL SGHKLSGLRFVLQDGAHHMVDSNEISFIRAGEGALKQALANGTLCIIEPIMSVEVIAPNEFQGT VFAGINRRHGVITGQDGIEDYFTLYADVPLNNMFGYSTELRSCTEGKGEYTMEYCRYQPCSPS TQEELINKYLEATGQLPVKKGKAKN (SEQ ID NO: 19), or has at least 80% sequence identity to SEQ ID NO: 19. In further embodiments, the fusion protein has at least 90% or 95% sequence identity to SEQ ID NO: 19. The sequence of SEQ ID NO: 19 comprises the N-terminally the HIS tag of SEQ ID NO: 9 that is fused via the linker of SEQ ID NO: 18 to the TAT sequence of SEQ ID NO: 7, followed by the linker of SEQ ID NO: 17 fusing the TAT sequence to the natural precursor protein of mouse Gfm1 as set forth in GenBank Accession No. NP_079272.4.In embodiments, the fusion protein further comprises C-terminally the HA tag of SEQ ID NO: 13. The fusion protein of SEQ ID NO: 19 comprising mouse EF-G1 is of particular relevance in experimental use.

The fusion protein is usable in the free acid or base form, or in the form of pharmaceutically acceptable salts, or as mixtures thereof. The term "pharmaceutically acceptable salt" refers to salts prepared from pharmaceutically acceptable non-toxic bases or acids. A pharmaceutically acceptable salt can be conveniently prepared from pharmaceutically acceptable non-toxic bases, including inorganic bases and organic bases, organic anions, organic cations, halides or alkaline. The term pharmaceutically acceptable salt includes alkali metal salts and addition salts of free acids or free bases. Suitable pharmaceutically acceptable base addition salts of the fusion proteins include metallic salts and organic salts.Preferred salts derived from inorganic bases include ammonium, calcium, magnesium, potassium and sodium salts. Salts derived from pharmaceutically acceptable organic non-toxic bases include salts of primary, secondary, and tertiary amines.

Another aspect of the invention relates to the fusion protein according to the invention for use as a medicament. The protein transduction domain with cell-penetrating properties offers the possibility to cross cell membranes. Coupling to a mitochondrial targeting sequence (MTS) provides localization in the subcellular mitochondrial compartment. Cleavage of the MTS inside the mitochondrial compartment leaves the mitochondrial elongation factor G1 peptide or protein in the mitochondrial matrix. This enables for a therapeutic use of the fusion protein.

The penetrating of cell membranes and deliverance of mitochondrial elongation factor G1 into mitochondria enables for a use in the treatment of a mitochondrial disease, for example by a mitochondrial protein replacement therapy.

A further aspect of the present invention relates to the fusion protein according to the invention for use in the treatment of a mitochondrial disease. The term "mitochondrial disease" as used herein refers to a group of disorders, which share as a common pathological feature defects in the mitochondrial oxidative phosphorylation system. This defect can be caused by disturbances in several processes including mutations in structural genes and mitochondrial tRNAs as well as affected mitochondrial DNA replication, transcription and translation. Damage to the mitochondria particularly causes energy shortage within tissue that consumes large amounts of energy such as the liver, muscles, brain, and the heart.

In preferred embodiments, the mitochondrial disease is selected from the group comprising mitochondrial elongation factor G1 deficiency and/or combined oxidative phosphorylation deficiency 1 (COXPD1). The term "combined oxidative phosphorylation deficiency" refers to a defect in the mitochondrial oxidative phosphorylation (OXPHOS) system involving more than one of the five OXPHOS complexes resulting in an autosomal recessive multisystem disorder with variable manifestations.

Defects in the mitochondrial elongation factor G1 (GFM1, EF-G1) can cause an enzymatic activity deficiency, which can be accompanied by a deficiency of the mitochondrial elongation factor G1 protein. Particularly, genetic alteration of the GFM1 gene resulting in EF-G1 defect can cause combined oxidative phosphorylation deficiency type 1 (COXPD1) (OMIM 609060). Combined oxidative phosphorylation deficiency-1 can be caused by homozygous or compound heterozygous mutation in the gene encoding the mitochondrial elongation factor G1 (GFM1, EF-G1) on human chromosome 3q25. Deficiency in mitochondrial elongation factor G1 and/or combined oxidative phosphorylation particularly can be due to Asn174Ser, Arg47Ter, Ser321Pro, Met496Arg, Arg250Trp, Arg671Cys, Leu607Ter or Leu398Pro substitution in the mitochondrial elongation factor G1 protein. The three letter code "Ter" denotes for a stop of the amino acid sequence. These allelic variants cause a severe disease usually associated with severe brain damage. Combined oxidative phosphorylation deficiency-1 can show prenatal manifestation and/or onset occurs at or soon after birth. Features can include growth retardation, microcephaly, hypertonicity, axial hypotonia, encephalopathy, cardiomyopathy, and liver dysfunction and cause premature death.

All diagnosed cases so far result in severe and early-fatal diseases. The majority of patients show very severe, early-onset multisytemic clinical phenotypes within the first year of life. The most frequent clinical presentation of children is (intaurine) growth retardation, developmental anomalies, muscular hypotonia and muscle weakness accompanied by encephalopathy, cardiomyopathy, optic atrophy, dysmorphy and hepatopathy. All patients showed significant reduction in activity levels in more than one OXPHOS complex, but with a normal OXPHOS complex II activity, the only OXPHOS complex that is exclusively encoded in the nuclear DNA.

In embodiments, the mitochondrial disease is selected from the group comprising encephalopathy particularly infantile encephalopathy, early infantile epileptic encephalopathy and hepatoencephalopathy, sudden infant death syndrome, seizures, mitochondrial myopathy, hepatopathy, cardiomyopathy, optic atrophy, hypoplasia of the vermis and severe pontine atrophy of the brainstem, liver failure with hypoglycemia and/or developmental delay resulting in a premature death, or is correlated with a prenatal manifestation such as intrauterine growth retardation.

The term "treatment" as used herein refers to alleviating at least one of the symptoms associated with the disease, decreasing disease-caused mortality, or decelerating, impeding or suspending the progress of the disease. The term "treatment" in the context of the intention does not refer to complete curing of the disease, as it does not change the mutated genetics causing the disease. A treatment can include compensating for an affected protein, for example by protein replacement.

In an embodiment, the fusion protein according to the invention is for use in mitochondrial protein replacement therapy. The term "protein replacement therapy" refers to a therapeutic approach for metabolic disorders wherein a deficient, defected or mutated protein is artificially manufactured, purified and administered to a patient, usually on a regular basis.

It was found that an EF-G1 protein transduction using the fusion protein of SEQ ID NO: 19 C-terminally fused to the HA tag of SEQ ID NO: 13 in EF-G1 deficient mouse embryonic fibroblasts effectively led to a mitochondrial localization of the transduced fusion protein. In addition, it was observed that the transduction with the fusion protein could complement EF-G1 deficiency and the resulted in a partial recovery of OXPHOS defects. Thus, it was found that administration of purified fusion protein advantageously was able replace or complement the deficient protein in vitro.

Successful TAT-mediated EF-G1 replacement therapy by injection of the fusion protein peptide also could be shown in vivo in mice with a brain-specific EF-G1 knock-out resulting in a forebrain specific EF-G1 deficiency. A partial recovery of EF-G1 protein levels was observed, and the treatment with fusion protein partially restored OXPHOS proteins in the EF-G1 knock-out mice. Further, no adverse effects on translation/transcription machinery, and also mitoribosome assembly, and in off-target tissues such as liver, heart and muscle was seen. Protein transduction in wild-type mice showed no lethality and no adverse effects on OXPHOS system, while protein levels of EF-G1 were increased.

The fusion protein for use as a medicament particularly in the treatment of a mitochondrial disease or in mitochondrial protein replacement therapy comprises a protein transduction domain, a mitochondrial targeting sequence and a mitochondrial elongation factor G1 peptide. In an embodiment, the mitochondrial elongation factor G1 peptide comprises the sequence of SEQ ID NO: 2, which refers to the enzymatic active part of human Gfm1, or has at least 80%, 90% or 95% sequence identity to SEQ ID NO: 2. In embodiments, the mitochondrial targeting sequence is selected from the group of SEQ ID NOs: 1, 3, 4 and 5, or has at least 80%, 90% or 95% sequence identity to these. In a preferred embodiment, the fusion protein comprises the MTS of SEQ ID NO: 1 and the EF-G1 peptide of SEQ ID NO: 2. A preferred protein transduction domain is a TAT peptide. Preferred TAT peptide sequences are selected from the group of SEQ ID NOs: 6 to8, or have at least 80%, 90% or 95% sequence identity to these. The protein transduction domain may be fused to the mitochondrial targeting sequence via a linker, preferably selected from SEQ ID NOs: 17 and 18. In embodiments, the fusion protein comprises at least one N-terminal and/or C-terminal tag selected from the group of SEQ ID NOs: 9 to 15, or a tag having at least 80%, 90% or 95% sequence identity to these. An N-terminal HIS tag such as of SEQ ID NOs: 9 or 10 may be fused to the TAT sequence of the fusion protein via a linker such as the linker of SEQ ID NO: 18. In embodiments, the HA tag of SEQ ID NO: 13 is fused C-terminally to the fusion protein. In embodiments, the fusion protein comprises a protein transduction domain being a TAT sequence, and the natural MTS of the human mitochondrial elongation factor G1 peptide. In a preferred embodiment, the fusion protein has the sequence of SEQ ID NO: 16 or has at least 80%, 90% or 95% sequence identity to SEQ ID NO: 16. In an embodiment, the fusion protein further comprises C-terminally the HA tag of SEQ ID NO: 13.

The fusion protein according to the invention can be used or included in a composition. Particularly for use as a medicament the fusion protein can be formulated as a pharmaceutical composition. A further aspect of the present invention hence relates to a pharmaceutical composition comprising as an active ingredient a fusion protein according to the invention, wherein the pharmaceutical composition is particularly for use in the treatment of a mitochondrial disease. In an embodiment, the pharmaceutical composition comprises: a) a fusion protein according to the invention as an active ingredient, and b) a pharmaceutically acceptable carrier.

Suitable pharmaceutically acceptable carriers include but are not limited to water, salt solutions such as NaCl, saline, buffered saline, alcohols, glycerol, ethanol, polyethylene glycols, hydroxymethylcellulose, or combinations thereof. The pharmaceutical preparations can, if desired, be mixed with auxiliary agents such as preservatives, stabilizers, wetting agents, emulsifiers, salts or buffers, which do not deleteriously react with the active peptide or interference with its activity. In embodiments, a water-soluble carrier suitable for intravenous administration is usable. The composition can be formulated in accordance with the routine procedures as a pharmaceutical composition adapted for administration to human beings. The compositions can be suitable for oral, dermal, rectal, topical, and parenteral administration. The compositions particularly can be suitable for parenteral administration, including subcutaneous, intramuscular, and intravenous administration. For example, in an embodiments, a composition for intravenous administration is a solution in sterile isotonic aqueous buffer.

A further aspect of the present invention relates to the use of a fusion protein according to the invention for the manufacture of a medicament, particularly for the manufacture of a medicament for the treatment of a mitochondrial disease.

A further aspect of the present invention relates to a method of treating a mitochondrial disease, the method comprising the step of administering to a subject a therapeutically effective amount of a fusion protein according to the invention. In an embodiment, the method of treating a mitochondrial disease is a mitochondrial protein replacement therapy.

Subjects include both human subjects and animal subjects, particularly mammalian subjects such as mice for medical and drug development purposes. The subject may include human subjects, and particularly may include neonate, infant or child subjects.

A fusion protein or a composition containing the same is administered in a therapeutically effective amount, the term "therapeutically effective amount" referring to a dosage amount that is sufficient to treat the disease, such as by ameliorating symptoms associated with the disease, preventing or delaying the onset of the disease, and/or also lessening the severity or frequency of symptoms of the disease. The treatment may be a continuous prolonged treatment, or include few time administrations for the treatment of an acute condition or during a crisis such as an infection.

The fusion protein, composition and method of the invention may be used to treat a subject suffering from or susceptible to a mitochondrial disease. The mitochondrial disorders particularly may be mitochondrial elongation factor G1 deficiency and/or combined oxidative phosphorylation deficiency 1 (COXPD1). Deficiency in mitochondrial elongation factor G1 and/or combined oxidative phosphorylation particularly can be due to Asn174Ser, Arg47Ter, Ser321Pro, Met496Arg, Arg250Trp, Arg671Cys, Leu607Ter or Leu398Pro substitution in the mitochondrial elongation factor G1 protein. In embodiments, the mitochondrial disease is selected from the group comprising encephalopathy particularly infantile encephalopathy, early infantile epileptic encephalopathy and hepatoencephalopathy, sudden infant death syndrome, seizures, mitochondrial myopathy, hepatopathy, cardiomyopathy, optic atrophy, hypoplasia of the vermis and severe pontine atrophy of the brainstem, liver failure with hypoglycemia and/or developmental delay resulting in a premature death, or is correlated with a prenatal manifestation such as intrauterine growth retardation.

The fusion protein for use in the manufacture of a medicament or in a method of treating a mitochondrial disease comprises a protein transduction domain, a mitochondrial targeting sequence and a mitochondrial elongation factor G1 peptide. In an embodiment, the mitochondrial elongation factor G1 peptide comprises the sequence of SEQ ID NO: 2, which refers to the enzymatic active part of human Gfm1, or has at least 80%, 90% or 95% sequence identity to SEQ ID NO: 2. In embodiments, the mitochondrial targeting sequence is selected from the group of SEQ ID NOs: 1, 3, 4 and 5, or has at least 80%, 90% or 95% sequence identity to these. In a preferred embodiment, the fusion protein comprises the MTS of SEQ ID NO: 1 and the EF-G1 peptide of SEQ ID NO: 2. A preferred protein transduction domain is a TAT peptide. Preferred TAT peptide sequences are selected from the group of SEQ ID NOs: 6 to8, or have at least 80%, 90% or 95% sequence identity to these. The protein transduction domain may be fused to the mitochondrial targeting sequence via a linker, preferably selected from SEQ ID NOs: 17 and 18. In embodiments, the fusion protein comprises at least one N-terminal and/or C-terminal tag selected from the group of SEQ ID NOs: 9 to 15, or a tag having at least 80%, 90% or 95% sequence identity to these. An N-terminal HIS tag such as of SEQ ID NOs: 9 or 10 may be fused to the TAT sequence of the fusion protein via a linker such as the linker of SEQ ID NO: 18. In embodiments, the HA tag of SEQ ID NO: 13 is fused C-terminally to the fusion protein. In embodiments, the fusion protein comprises a protein transduction domain being a TAT sequence, and the natural MTS of the human mitochondrial elongation factor G1 peptide. In a preferred embodiment, the fusion protein has the sequence of SEQ ID NO: 16 or has at least 80%, 90% or 95% sequence identity to SEQ ID NO: 16. In an embodiment, the fusion protein further comprises C-terminally the HA tag of SEQ ID NO: 13.

Another aspect of the invention relates to a viral or non-viral vector system, comprising a nucleic acid sequence encoding for a fusion protein according to the invention. As used herein, the term "vector" refers to discrete elements that are used to introduce heterologous DNA into cells for either expression or replication thereof. Selection and use of such vehicles are well known within the skill of the artisan.

Suitable vector systems in the form of viral vectors such as retroviral, adenoviral vectors or adeno-associated vectors are known to a person skilled in the art. Also non-viral vectors and delivery systems may be used. Non-viral vectors may be selected from liposomes, polymers such as polyethylene imines or dendrimers, peptide-DNA complexes or the administration of naked DNA by physical methods such as electroporation, or "gene gun". A simple method of non-viral transfection is the injection of naked DNA.

The viral or non-viral vector system may be an expression vector. As used herein, the term "expression vector" particularly refers to a vector capable of effecting protein expression in a host cell. An expression vector may be a recombinant DNA construct, such as a plasmid, that, upon introduction into an appropriate host cell, results in expression of a cloned DNA. Suitable vectors have regulatory sequences such as promoters and the like as desired for expression in prokaryotes or in eukaryotic cells. Appropriate expression vectors are well known to those of skill in the art. An example is the vector pTriEX-HTNC.

A pharmaceutical composition may comprise as an active ingredient a viral or non-viral vector system such as an expression vector comprising a nucleic acid sequence encoding for a fusion protein according to the invention, wherein the pharmaceutical composition is particularly for use in the treatment of a mitochondrial disease. In an embodiment, the pharmaceutical composition comprises: a) a viral or non-viral vector system such as an expression vector comprising a nucleic acid sequence encoding for a fusion protein according to the invention as an active ingredient, and b) a pharmaceutically acceptable carrier. The viral or non-viral vector system such as an expression vector according to the invention particularly is usable in the treatment of a mitochondrial disease. In an embodiment, the viral or non-viral vector system such as an expression vector according to the invention is for use in mitochondrial protein replacement therapy. A further aspect of the present invention relates to a method of treating a mitochondrial disease, the method comprising the step of administering to a subject a therapeutically effective amount of viral or non-viral vector system comprising a nucleic acid sequence encoding for a fusion protein according to the invention.

Unless otherwise defined, the technical and scientific terms used herein have the same meaning as commonly understood by one of ordinary skill in the art to which this invention belongs.

The Examples, which follow serve to illustrate the invention in more detail but do not constitute a limitation thereof.

The figures show:

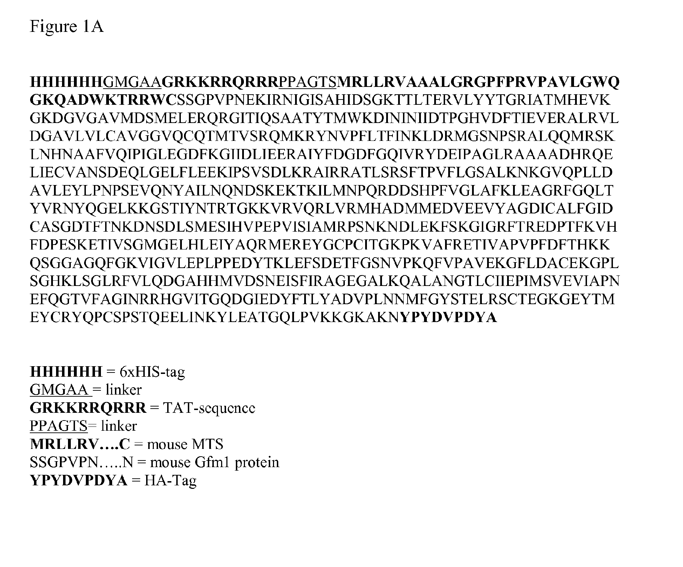

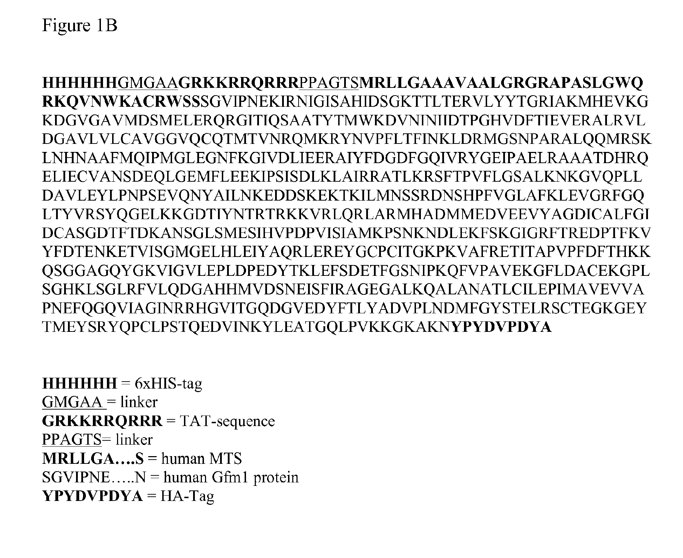

- Figure 1:: the amino acid sequence of fusion proteins. Figure 1A shows the amino acid sequence of a mouse TAT-GFM1 fusion protein according to an embodiment, while Figure 1B shows the amino acid sequence of a human TAT-GFM1 fusion protein according to an embodiment.

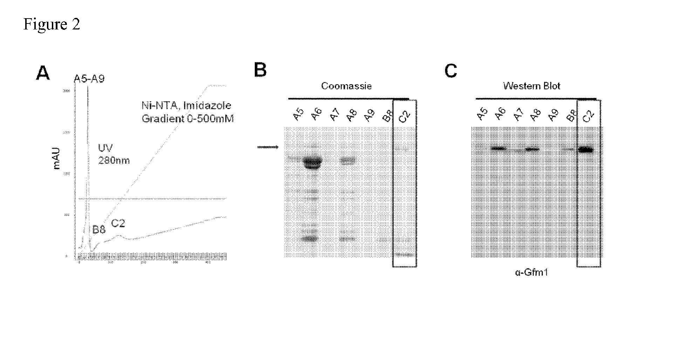

- Figure 2:: Ni-NTA-purification of a TAT-GFM1 fusion protein according to an embodiment (example 2). Shown are a representative chromatogram (A), coomassie-stained gel (B) and immunoblot using an antibody directed against GFM1 (C). An arrow depicts the construct. The selected fraction C2 is indicated by a box.

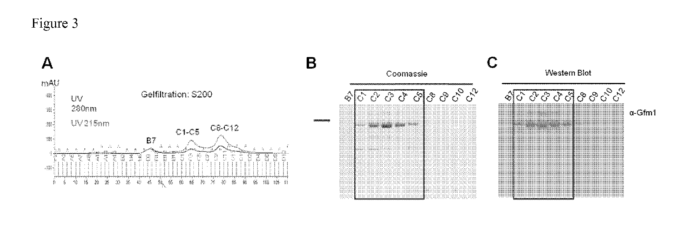

- Figure 3:: Gel-filtration of the Ni-NTA-purified TAT-GFM1 construct. Shown are a representative chromatogram (A), a coomassie-stained gel (B) and an immunoblot using an antibody directed against GFM1 (C). An arrow depicts the fusion protein. The selected fractions C1-C5 are indicated by a box.

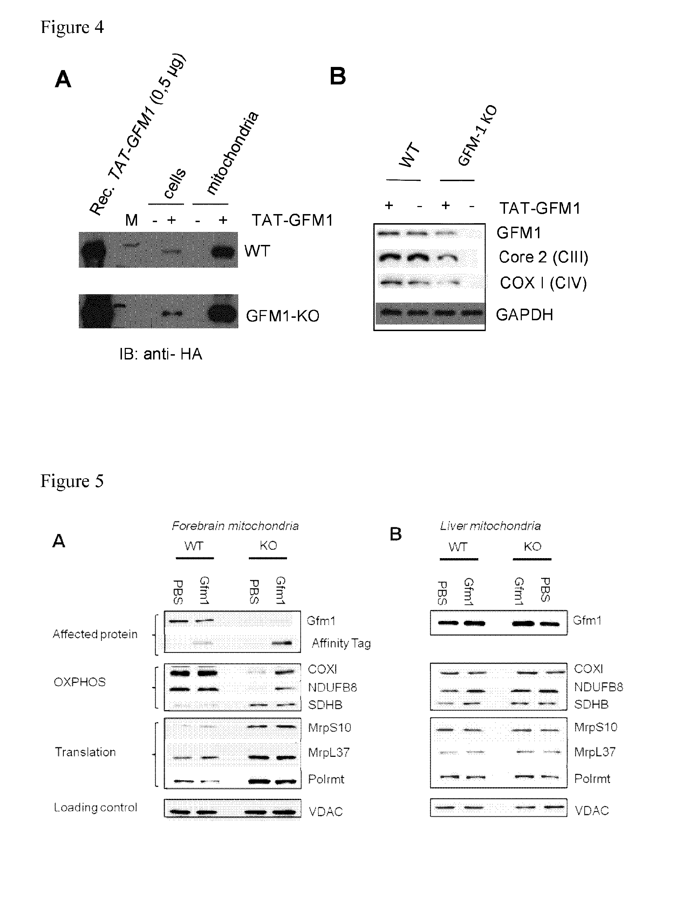

- Figure 4:: Protein transduction with the TAT-GFM1 fusion protein. (A) shows immunoblottings of untreated and treated wild-type (WT) and GFM1 knock-out (GFM1-KO) MEFs. Cell and mitochondrial extracts were probed to verify the mitochondrial localization of the transduced TAT-GFM1 fusion protein. (B) shows transduced wild-type (WT) and GFM1 knock-out (GFM1-KO) MEFs which were either transduced with the TAT-GFM1 fusion protein or not transduced and assessed for GFM1 and levels of OXPHOS proteins cytochrome b-c1 complex subunit 2 (Core 2, CIII) and cytochrome c oxidase I (COX I, CIV). GAPDH was used as standard.
- Figure 5:: Protein transduction with TAT-GFM1 fusion protein in wild-type- (WT) and GFM1-knock-out (KO) mice. (A) Mitochondria isolated from brain were probed for the presence of endogenous GFM1 as well as for the recombinant protein using the anti-HA antibody. In addition, OXPHOS marker proteins and markers for mitochondrial translation were assessed. (B) The non-affected tissue liver was analyzed to assess adverse effects of the transduction. The same markers as in (A) were used.

Example 1: Construction of a bacterial expression vector expressing a TAT-GFM1 fusion construct

The cDNA coding for mouse GFM1 was amplied from cDNA prepared from mouse embryonic fibroblasts (MEFs). The gene was amplified via PCR using the primers 5'-AGCATGACTAGTATGCGGCTGCTGCGGGTCGC-3' set forth in SEQ ID NO: 20 (forward) and 5'-CATGCTCTCGAGTCAAGCGTAATCTGGTACGTCGTATGGGTAGTTCTTGGCTTTCCCTTTT T-3' SEQ ID NO: 21 (reverse) which introduced a C-terminal hemagglutinin (HA) tag as well as restriction sites for SpeI and XhoI. The primers were designed to amplify the cDNA for the mouse EF-G1 precursor protein including its natural MTS. The resulting fragment was cloned into the vector pTriEX-HTNC (plasmid No. 13763, Addgene), which harboured already a 6xHis-Tag and a TAT sequence to yield the final fusion protein. The PCR fragment was cloned downstream of the TAT sequence into the vector. The resulting construct includes an N-terminal the HIS tag, fused via a linker to the TAT sequence, followed by a linker fusing the TAT sequence to the natural precursor protein of mouse GMF-1 including its natural mitochondrial targeting sequence and the enzymatic active GMF-1protein, followed by a C-terminal the HA tag. These correspond to the amino acid sequences of SEQ ID NO: 19 that is C-terminally fused to SEQ ID NO: 13. The amino acid sequence of the construct is depicted in Figure 1A and is set forth in SEQ ID NO: 22. The construct is in the following examples referred to as "TAT-GFM1 fusion protein" or shortly "TAT-GFM1".

The cDNA coding for human GFM1 was amplied from cDNA prepared from a HeLa cell line. The human gene was amplified via PCR using the forward and reverse primers 5'-AGCATGACTAGTATGAGACTCCTGGGAGCTGCAG-3' of SEQ ID NO: 23 and 5'-TCATGCCTCGAGTTAAGCGTAATCTGGTACGTCGTATGGGTAGTTCTTGGCTTTTCCTTTTT -3' of SEQ ID NO: 24, respectively, designed to amplify the cDNA for the human EF-G1 precursor protein including its natural MTS and cloned into the vector pTriEX-HTNC. The resulting human construct is depicted in Figure 1B and is set forth in SEQ ID NO: 25.

### Example 2: Protein expression and purification of TAT-GFM1 fusion protein

The mouse construct of example 1 was transformed into E. coli BL21 (DE3) cells. Transformed bacterial cells were inoculated into 100 ml LB (lysogeny broth) media containing 1mM Ampicilin (LB-Amp). The culture was incubated overnight at 37°C at 165 rpm. The next day, 5-101 of LB-Amp was inoculated with the preculture in a dilution of 1:75. The cultures were incubated at 37 °C, 165 rpm until the optimal density (OD), which was measured against clean LB, reached 0.4, when protein expression was induced by addition of 0.5 mM IPTG (end-concentration). The cultures were shifted to 18°C and 165 rpm and incubated overnight (16 h). The bacterial pellet was collected by centrifugation (Beckmann centrifuge, JLA 8.1000 rotor, 4000 rpm, 4°C, 40 min). The pellet was resuspended in a mixture of 15 ml lysis buffer and 1 1 culture (lysis buffer: 150 mM NaCl, 50 mM Tris/HCl, pH 7.4, 2 mM ß-mercaptoethanol, 100 µM PMSF (phenylmethanesulfonyl fluoride) or protease inhibitor mix). The suspension of pellet in lysis buffer was stored at -80°C.

The frozen suspension was thawn on ice and sonicated at 4°C using the Branson Sonifier 250 (1x2min, 80% duty cycle, 8 output control; 3x30 sec, 80% duty cycle, 8 output control). The sonicated suspension was centrifuges (Beckman centrifuge, 1 hr, 4°C, 20.000xg, rotor JA 25.50) and the supernatant was used further on for the purification by nitrilotriacetic acid (Ni-NTA) chromatography utilizing the Histine-affinity tag in the construct. The protein purification was carried out on an ÄKTA purifier system using commercially available Ni-NTA-matrix (30 ml volume). The purification protocol employed a gradient from 100% equilibration buffer (150 mM NaCl, 50 mM Tri/HCl ph 7.4, 2 mM ß-mercaptoethanol, 20 mM imidazole) to 100% elution buffer (150 mM NaCl, 50 mM Tri/HCl ph 7.4, 2 mM ß-mercaptoethanol, 500 mM Imidazole) in 400 ml total volume.

A representative run is shown in figure 2A. Fractions were analyzed by coomassie-staing as is shown in figure 2B. Figure 2C shows an immunblotting using an antibody directed against the TAT-GFM1 fusion protein (Abcam (ab107496)). An arrow depicts the fusion protein. The selected fraction C2 is indicated by a box. Positive fractions were pooled and concentrated to about 2 mL with Amicon (30 kDa cut off).

For further purification, the pooled fractions were applied to gel filtration on the Äkta purifier (column material S200; 26/60) and peaks were analyzed as before by coomassie staining and immunoblotting. Representative images of the chromatogram, stained gel and immunoblot are depicted in figure 3. An arrow depicts the fusion protein. The selected fractions C1 to C5 are indicated by a box. Selected fractions were concentration to 2 mL with Amicon (30 kDa cut off) and stored in 50 µl aliquots at - 80°C at a concentration of 5-10 µg/l.

### Example 3: Treatment of wild-type and GFM1-knockout cells with a TAT-GFM1 fusion protein

To determine the effect of protein transduction of the purified TAT-GFM1 fusion protein, wild-type mouse embryonic fibroblasts (MEFs) and GFM1-knockout MEFs were transduced with the purified TAT-GFM1 fusion protein of example 2. For generating GFM1-knockout MEFs, MEFs were isolated from a mouse carrying homozygous floxed alleles of GFM1. The resulting MEFs were then in cell culture cultivated and the floxed sequence was deleted by transfection with a Cre-expressing vector to generate the knock-out cell line.

The purified fusion protein of example 2 was dialyzed against PBS (scienova xpress MicroDialyzer (1 h, 4°C)). The cells were seeded at a density of 10⁵ in 6-well dishes so that a confluency of about 50% was obtained the next day. The cells were washed 3 times with PBS. The TAT-GFM1 fusion protein was diluted with a 1:1 mixture of PBS:DMEM (Dulbecco's Modified Eagle's Medium) to 20 µg/ml and a total of 40 µg was added to each well. The cells were incubated with the fusion protein mixture for 5 h, then the solution was removed and the cells were washed 3 times with PBS and allowed to recover and proliferate in normal DMEM for 48 h prior to immunohistochemical labeling and protein extraction.

The mitochondrial localization of the fusion protein TAT-GFM1 was probed with immunohistochemical labeling of fixed cells on a slide using an antibody against the hemagglutinin (HA) tag (anti-HA antibody, abcam) to detect the transduced protein and using an antibody against the translocase of outer mitochondrial membranes (TOM), a multiprotein complex at the outer membrane of mitochondria (anti-TOM20, abcam) to stain mitochondria. It could be seen that the staining for both TOM20 marking the mitochondrial compartment and the staining for HA marking the transduced protein overlapped indicating co-localization and hence mitochondrial localization of the transduced protein. Importantly, we did not detect HA-signal in the cytoplasma suggesting that all of the transduced protein is localized in the designated mitochondrial compartment.

Cells and isolated mitochondria were lysed and proteins were extracted from further dishes of the cultured wild-type and GFM1-knockout MEFs using RIPA Buffer (25mM Tris-HCl (pH 7.6), 150mM NaCl, 1% NP-40, 1% sodium deoxycholate, 0.1% SDS). RIPA extracts of total cells or isolated mitochondria were probed with anti-HA antibody for the transduced protein to verify the mitochondrial localization of the fusion protein. Figure 4A shows immunoblottings using anti-HA antibody of cell and mitochondrial protein extracts of wild-type (WT) and GFM1 knock-out (GFM1-KO) MEFs untreated or treated with TAT-GFM1 fusion protein for 5 h. As can be taken from Figure 4A, the TAT-GFM1 fusion protein was detected in extracts of cell proteins and was found exclusively localized in extracts of isolated mitochondrial proteins. This finding verifies the mitochondrial localization of the transduced TAT-GFM1 fusion protein.

Proteins of the oxidative phosphorylation (OXPHOS), which show a defect in GFM1-deficient cells, were also probed by immunoblotting to assess the recovery of these proteins upon transduction with the TAT-GFM1 fusion protein. Figure 4B immunoblots of the wild-type and GFM1 knock-out MEFs which were either transduced with the TAT-GFM1 fusion protein or not transduced and assessed for GFM1 and levels of the OXPHOS proteins cytochrome b-c1 complex subunit 2 (Core 2) of complex III and cytochrome c oxidase I (COX I) of complex IV. Glyceraldehyde 3-phosphate dehydrogenase (GAPDH) was used as standard. As can be seen from Figure 4B, upon transduction with the TAT-GFM1 fusion protein the protein levels of GFM1, Core 2 and COX I increased relieving the deficiency. It could be further seen that the transduction of wild-type MEFs did not result in adverse effects indicating that GFM1-transduction does not cause harm to non-affected cells.

The OXPHOS defect is the disease-causing mechanism in patients. Hence, the partial recovery of OXPHOS upon GFM1-tranduction indicated a therapeutic potential of the fusion protein.

### Example 4: Treatment of mice with TAT-GFM1 fusion protein

To assess the in vivo effect of the TAT-GFM1 fusion protein, a mouse-model of GFM1-defiency restricted to the brain was used. A brain-specific conditional knock-out of mitochondrial elongation factor G1 (EF-G1 KO) was generated by crossing mice homozygous for a floxed EF-G1 allele with mice expressing Cre recombinase in a brain-specific manner expressed from a CamKIIα-promotor (CamKIIa (Calmodulin-Dependent Protein Kinase II)-Cre). The resulting brain-specific loss of EF-G1 caused a dramatic decrease in life expectancy with no animal surviving past the age of 6 months associated with early-onset motility and memory deficits.

The purified TAT-GFM1 fusion protein was dialyzed against PBS before intraperitoneal injection of 20 µg/g bodyweight into wild-type (n=5) and GFM1-knock-out (n=5) mice. Control mice of wild-type (n=5) and GFM1-knockout (n=5) mice received intraperitoneal injection of PBS. The mice were sacrificed 5 days after injection and tissue and mitochondria were isolated an analyzed. Mitochondria were isolated from forebrain and liver of wild-type and GFM1-knockout mice, lysed and proteins were extracted using RIPA Buffer.

Mitochondria isolated from brain being the affected tissue were probed by immunoblotting to assess for their GFM1-content to (a) determine if the TAT-GFM1 protein reached the brain, (b) is able to reside in mitochondria and (c) to affect OXPHOS system. The Figure 5A shows immunoblots depicting the protein level of different proteins after protein transduction with TAT-GFM1 fusion protein (Gfm1) in wild-type- (WT) and GFM1-knockout (KO) mice and controls (PBS). Protein levels of the mitochondrial outer membrane protein voltage-dependent anion channel (VDAC) were used as loading control.

Forebrain mitochondria were probed for the presence of endogenous GFM1 (Gfm1) using anti-GFM1 antibody (Abcam (ab 107496)), as well as specifically for the recombinant protein (Affinity tag) using the anti-HA antibody. As can be seen in Figure 5A, upon transduction with the TAT-GFM1 fusion protein the protein levels of Gfm1 strongly increased compared to the controls in in wild-type- (WT) and GFM1 knock-out (KO) mice. In addition, OXPHOS marker proteins cytochrome c oxidase I (COX I), subunit NDUFB8 of OXPHOS complex I (NDUFB8), and subunit b of succinate dehydrogenase (SDHB) were used to assess the therapeutic effect of the transduction. As can be seen in Figure 5A, the OXPHOS markers proteins, which showed a clear deficiency in GFM1 KO tissue, recovered after protein transduction resulting in increased protein levels and hence a partial recovery of OXPHOS.

These findings show that GFM1 is present in brain mitochondria in transduced animals and partially rescues the OXPHOS defect as evidenced by partially increased OXPHOS marker protein comparing PBS and TAT-GFM1 injected animals.

Moreover, protein levels of small mitoribosomal subunit 10 (MrpS10), large mitoribosomal subunit 37 (MrpL37), and mitochondrial RNA polymerase (Polrmt) as markers for mitochondrial translation were employed to assess if the transduction has an adverse effect on mitochondrial processes. As can be seen in Figure 5A, no significant change in protein levels of mitochondrial translation markers was observed upon transduction with the TAT-GFM1 fusion protein in either wild-type- (WT) or GFM1 knock-out (KO) mice compared to the controls. This shows that in neither wild-type nor knock-out tissues did the protein transduction affect mitochondrial processes such as the mitochondrial translation machinery indicated by unchanged marker levels in the immunoblotting. Hence, no adverse effect were observed in treated versus untreated wild-type animals.

Mitochondria of the liver were assessed as control to analyze the effect of the protein transduction in non-affected tissue. The Figure 5B shows immunoblots of the liver mitochondria depicting the protein level of Gfm1, and the same OXPHOS proteins and translation markers after protein transduction with TAT-GFM1 fusion protein (Gfm1) in wild-type- (WT) and GFM1-knock-out (KO) mice and controls (PBS). Protein levels of VDAC were used as loading control. As can be seen in Figure 4B, Gfm1 levels were slightly increased, while no significant alterations in OXPHOS and translation marker protein levels was seen upon transduction with the TAT-GFM1 fusion protein in liver mitochondria. Again, no adverse affects were observed when the effect of the protein transduction in non-affected tissues such as liver were assessed.

In summary, these findings indicate that the TAT-EF-G1 fusion protein penetrates the blood-brain barrier and in a single injection partially relieves the protein deficiency and ameliorates the OXPHOS defect.

## Claims

1. A fusion protein comprising
a) a protein transduction domain,
b) a mitochondrial targeting sequence, and
c) a mitochondrial elongation factor G1 peptide.

2. The fusion protein according to claim 1, wherein the mitochondrial elongation factor G1 peptide is a human or mouse Gfm1 protein.

3. The fusion protein according to claim 1 or 2, wherein the mitochondrial elongation factor G1 peptide comprises the following sequence SGVIPNEKIRNIGISAHIDSGKTTLTERVLYYTGRIAKMHEVKGKDGVGAVMDSMELERQRGI TIQSAATYTMWKDVNINIIDTPGHVDFTIEVERALRVLDGAVLVLCAVGGVQCQTMTVNRQ MKRYNVPFLTFINKLDRMGSNPARALQQMRSKLNHNAAFMQIPMGLEGNFKGIVDLIEERAI YFDGDFGQIVRYGEIPAELRAAATDHRQELIECVANSDEQLGEMFLEEKIPSISDLKLAIRRATL KRSFTPVFLGSALKNKGVQPLLDAVLEYLPNPSEVQNYAILNKEDDSKEKTKILMNSSRDNSH PFVGLAFKLEVGRFGQLTYVRSYQGELKKGDTIYNTRTRKKVRLQRLARMHADMMEDVEE VYAGDICALFGIDCASGDTFTDKANSGLSMESIHVPDPVISIAMKPSNKNDLEKFSKGIGRFTR EDPTFKVYFDTENKETVISGMGELHLEIYAQRLEREYGCPCITGKPKVAFRETITAPVPFDFTH KKQSGGAGQYGKVIGVLEPLDPEDYTKLEFSDETFGSNIPKQFVPAVEKGFLDACEKGPLSGH KLSGLRFVLQDGAHHMVDSNEISFIRAGEGALKQALANATLCILEPIMAVEVVAPNEFQGQVI AGINRRHGVITGQDGVEDYFTLYADVPLNDMFGYSTELRSCTEGKGEYTMEYSRYQPCLPST QEDVINKYLEATGQLPVKKGKAKN (SEQ ID NO: 2), or has at least 80% sequence identity to SEQ ID NO: 2.

4. The fusion protein according to any one of claims 1 to 3, wherein the mitochondrial targeting sequence is selected from the group comprising MRLLGAAAVAALGRGRAPASLGWQRKQVNWKACRWSS (SEQ ID NO: 1), MSVLTPLLLRGLTGSARRLPVPRAK (SEQ ID NO: 3), MLSRAVCGTSRQLAPVLGYLGSRQ (SEQ ID NO: 4) and/or MAFLRSMWGVLSALGRSGA (SEQ ID NO: 5), or having at least 80% sequence identity to SEQ ID NOs: 1, and 3 to 5.

5. The fusion protein according to any one of claims 1 to 4, wherein the protein transduction domain is a TAT sequence selected from the group comprising YGRKKRRQRRR (SEQ ID NO: 6), GRKKRRQRRR (SEQ ID NO: 7) and/or GRKKRRQRRRPQ (SEQ ID NO: 8), or has at least 80% sequence identity to SEQ ID NOs: 6 to 8.

6. The fusion protein according to any one of claims 1 to 5, wherein the fusion protein comprises at least one N-terminal and/or C-terminal tag selected from the group comprising HHHHHH (SEQ ID NO: 9), HHHHHHHHHH (SEQ ID NO: 10), AWRHPQFGG (SEQ ID NO: 11), WSHPQFEK (SEQ ID NO: 12), YPYDVPDYA (SEQ ID NO: 13), EQKLISEEDL (SEQ ID NO: 14) and/or DYKDDDDK (SEQ ID NO: 15), or has at least 80% sequence identity to SEQ ID NOs: 9 to 15.

7. The fusion protein according to any one of claims 1 to 6, wherein the fusion protein has the following sequence HHHHHHGMGAAGRKKRRQRRRPPAGTSMRLLGAAAVAALGRGRAPASLGWQRKQVNWK ACRWSSSGVIPNEKIRNIGISAHIDSGKTTLTERVLYYTGRIAKMHEVKGKDGVGAVMDSMEL ERQRGITIQSAATYTMWKDVNINIIDTPGHVDFTIEVERALRVLDGAVLVLCAVGGVQCQTM TVNRQMKRYNVPFLTFINKLDRMGSNPARALQQMRSKLNHNAAFMQIPMGLEGNFKGIVDL IEERAIYFDGDFGQIVRYGEIPAELRAAATDHRQELIECVANSDEQLGEMFLEEKIPSISDLKLAI RRATLKRSFTPVFLGSALKNKGVQPLLDAVLEYLPNPSEVQNYAILNKEDDSKEKTKILMNSS RDNSHPFVGLAFKLEVGRFGQLTYVRSYQGELKKGDTIYNTRTRKKVRLQRLARMHADMM EDVEEVYAGDICALFGIDCASGDTFTDKANSGLSMESIHVPDPVISIAMKPSNKNDLEKFSKGI GRFTREDPTFKVYFDTENKETVISGMGELHLEIYAQRLEREYGCPCITGKPKVAFRETITAPVP FDFTHKKQSGGAGQYGKVIGVLEPLDPEDYTKLEFSDETFGSNIPKQFVPAVEKGFLDACEKG PLSGHKLSGLRFVLQDGAHHMVDSNEISFIRAGEGALKQALANATLCILEPIMAVEVVAPNEF QGQVIAGINRRHGVITGQDGVEDYFTLYADVPLNDMFGYSTELRSCTEGKGEYTMEYSRYQP CLPSTQEDVINKYLEATGQLPVKKGKAKN (SEQ ID NO: 16), or has at least 80% sequence identity to SEQ ID NO: 16.

8. The fusion protein according to any one of claims 1 to 7, for use as a medicament.

9. The fusion protein according to any one of claims 1 to 7, for use in the treatment of a mitochondrial disease.

10. The fusion protein for use according to claim 9, wherein the mitochondrial disease is selected from the group comprising mitochondrial elongation factor G1 deficiency and/or combined oxidative phosphorylation deficiency, particularly due to Asn174Ser, Arg47Ter, Ser321Pro, Met496Arg, Arg250Trp, Arg671 Cys, Leu607Ter or Leu398Pro substitution in the mitochondrial elongation factor G1 protein.

11. The fusion protein for use according to claim 9 or 10, wherein the mitochondrial disease is selected from the group comprising encephalopathy particularly infantile encephalopathy, early infantile epileptic encephalopathy and hepatoencephalopathy, sudden infant death syndrome, seizures, mitochondrial myopathy, hepatopathy, cardiomyopathy, optic atrophy, hypoplasia of the vermis and severe pontine atrophy of the brainstem, liver failure with hypoglycemia and/or developmental delay resulting in a premature death, or is correlated with a prenatal manifestation such as intrauterine growth retardation.

12. The fusion protein for use according to any one of claims 9 to 11, wherein the treatment comprises a mitochondrial protein replacement therapy.

13. A pharmaceutical composition comprising as an active ingredient a fusion protein according to any one of claims 1 to 7, wherein the pharmaceutical composition is particularly for use in the treatment of a mitochondrial disease.

14. Use of a fusion protein according to any one of claims 1 to 7 for the manufacture of a medicament, particularly for the manufacture of a medicament for the treatment of a mitochondrial disease.

15. A method of treating a mitochondrial disease, the method comprising the step of administering to a subject a therapeutically effective amount of a fusion protein according to any one of claims 1 to 7.

16. A viral or non-viral vector system, comprising a nucleic acid sequence encoding for a fusion protein according to any one of claims 1 to 7.
